(19) Europäisches Patentamt
European Patent Office
Office européen des brevets

(11) **EP 2 085 092 A1**

(12) **EUROPEAN PATENT APPLICATION**

(43) Date of publication:
**05.08.2009 Bulletin 2009/32**

(51) Int Cl.:
**A61K 35/76** (2006.01)

(21) Application number: **08001643.9**

(22) Date of filing: **29.01.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Bayer Schering Pharma Aktiengesellschaft**
**13353 Berlin (DE)**

(72) Inventors:
• **Pühler, Florian**
  **14089 Berlin (DE)**
• **Beier, Rudolf**
  **13342 Berlin (DE)**

(74) Representative: **Weiss, Wolfgang et al**
**Weickmann & Weickmann**
**Patentanwälte**
**Postfach 86 08 20**
**81635 München (DE)**

(54) **Attenuated oncolytic paramyxoviruses encoding avian cytokines**

(57)    The invention refers to a recombinant oncolytic RNA Newcastle Disease Virus for the treatment of a proliferative disease, comprising at least one transgene coding for an avian cytokine, wherein the recombinant oncolytic RNA Newcastle Disease Virus is obtainable from a velogenic or mesogenic oncolytic RNA Newcastle Disease Virus. Virus-mediated expression of the cytokine in the natural host cells leads to a reduced pathogenicity of the virus for avian species. Furthermore the virus genome can encode binding proteins, prodrug-converting enzymes or/and proteases. The selective expression of these molecules in virus-infected tumor cells increases the anti-tumor effect of the virus.

**Figure 4**

EP 2 085 092 A1

**Description**

**Introduction**

The present invention refers to a recombinant oncolytic RNA Newcastle Disease Virus comprising at least one transgene coding for an avian cytokine, wherein the recombinant oncolytic RNA Newcastle Disease Virus is obtainable from a velogenic or mesogenic oncolytic RNA Newcastle Disease Virus. Virus-mediated expression of the cytokine in the natural host cells leads to a reduced pathogenicity of the virus for avian species.

The virus in the present invention is suitable for the treatment of diseases, especially for oncolytic tumor treatment. Recombinant viruses are produced that encode an avian cytokine, wherein the pathogenicity of the virus is reduced for an avian species leading to a diminished environmental toxicity of the virus. The oncolytic activity of the virus is not impaired by the described method of pathogenicity reduction. The virus genome may encode additional therapeutic transgenes, preferably binding proteins (antibodies, ankyrin repeat molecules, peptides etc.), prodrug-converting enzymes or/and proteases. The activity of these binding proteins, prodrug-converting enzymes and/or proteases increases the anti-tumor effect of the virus. Further the invention describes manufacture and the use of such modified viruses for treatment of cancer.

**Description of the State of the Art**

**Newcastle Disease Virus**

Oncolytic viruses in general for the treatment of tumors are reviewed in Chiocca (2002). Newcastle Disease Virus (NDV) has been used as an experimental therapeutic agent for more than 40 years and is reviewed by Sinkovics and Horvath (2000). The Newcastle Disease Virus in general is described in the book by Alexander (1988). NDV strain PV701 is being developed as an anticancer treatment for glioblastoma (Lorence et al., 2003). The NDV strain MTH68 has been used as an experimental cancer treatment and has been administered to humans for more than 30 years (Csatary et al., 2004).

In the paper by Stojdl et al. (2003) it is described that in the range of 80% of all tested tumor cell lines, there is a defect in the interferon response following infection with Vesicular Stomatitis Virus (VSV). It may be assumed that a similar percentage of tumor cell lines will be susceptible to infection with NDV because both VSV and NDV are members of the order mononegavirales. It has also been shown that the mechanism of selective replication of NDV in tumor cells is based on a defect in the cellular interferon response against the virus (see e.g. US: 20030044384).

Paramyxoviruses contain single-stranded RNA genomes of negative polarity having genomes of 15-19 kb in length (wild-type) and the genomes contain 6-10 genes. The viral envelope is formed by the surface glycoproteins and a membrane part derived from the host cell. The surface glycoproteins (F and HN or H or G) mediate entry and exit of the virus from the host cell. The nucleocapsid is inside the envelope and contains the RNA genome and the nucleocapsid protein (NP), phospho- (P) and large (L) proteins responsible for intercellular virus transcription and replication. The matrix (M) protein connects the viral envelope and the nucleocapsid. In addition to these genes encoding structural proteins, Paramyxoviridae may contain "accessory" genes which may be additional transcriptional units interspersed with the genes mentioned above. The accessory genes are mostly ORFs that overlap with the P gene transcriptional unit. A comprehensive description of paramyxoviridae can be found in (Lamb, 2001).

NDV is the prototypic member of the genus Avulavirus in the family Paramyxoviridae belonging to the order Mononegavirales. The viral genome is a single-stranded negative-sense RNA coding for six major proteins: the nucleocapsid protein (NP), phosphoprotein (P), matrix protein (M), fusion protein (F), hemagglutinin protein (HN), and the polymerase protein (L). By editing of the P protein mRNA, one or two additional proteins, V (and W), are translated.

NDV is in detail characterized in Alexander (1988) and Lamb (2001).

**Newcastle Disease virus as an avian pathogen**

NDV strains are classified on their pathogenicity for chicken as velogenic strains (highly virulent) leading to acute lethal infection of chicken of all ages, mesogenic isolates (intermediate virulence) that are only lethal in young chicks, and lentogenic strains (nonvirulent) manifested in a mild or unapparent form of the disease. Classification of NDV isolates in velogen, mesogen or lentogen is determined by the mean death time (MDT) of the chicken embryo in 9 day-old embryonated eggs after inocculation with the minimum lethal dose to kill the embryo. One of the determinants of NDV virulence seems to be the cleavage site of the precursor F protein.

The first outbreak of NDV were observed in the year 1926 in Newcastle-upon-Tyne, England and in Java, Indonesia. Three NDV panzootics have been described since the first appearance of the disease.

NDV is primarily transmitted by aerosols or large droplets that are inhaled by susceptible birds. During the course of infection new infectious virus particles will be shed from the infected respiratory tract or excreted in the feces. By ingestion of this virus-containing material by healthy birds, new infections can be established and virus-spreading from one bird to another can be maintained.

NDV as an avian pathogen is described in detail in Alexander (1997), Diseases of Poultry.

**Newcastle Disease Vaccination**

NDV as a threat for poultry farming and the need for vaccination has been already recognised at the beginning of the last century. Iyer and Dobson have performed studies in the 1930s on the attenuation of virulent NDV strains, leading to the development of some mesogenic NDV vaccine strains. The development of live vaccines was moved forward by the establishment of the lentogenic vaccine strains Hitchner B1 and LaSota, which are now the most widely used NDV vaccines. Further vaccination strategies includes the use of inactivated NDV vaccines (e.g. by formaline, β-propiolactone), given together with a carrier adjuvant (aluminum hydroxide, oil emulsion).

The history and the actual state of development of NDV vaccines is depicted in detail in Alexander (1997) Diseases of Poultry.

Next generation vaccines for veterinary use based on recombinant NDV generated by reverse genetic technology are reviewed in Huang et al. (2003) and recombinant NDV vaccines are described in the following patents.

US 6,699,479 B1 describes a Newcastle disease virus (NDV) mutant that expresses its V protein at a reduced level and is used for vaccination of embryos before hatch.

US 2004/0043035 relates to a recombinant NDV mutant that is not able to express an immunodominant epitope of the nucleoprotein (NP) and is suited as a marker vaccine strain.

US 2003/0224017 describes a reverse genetic system for NDV for the production of a recombinant NDV vaccine. This system allows the expresssion of transgenes, e.g. avian cytokines (chicken IL-2, chicken IL-4) from the NDV genome to generate NDV vaccine strains.

EP 1 300 157 relates to an attenuated mutant Newcastle disease virus strain suitable for in ovo vaccination of avian species comprising a mutation in the gene sequences encoding the HN and/or F glycoproteins.

US 6,719,979 relates to a process for generating infectious Newcastle disease virus (NDV) entirely from cloned full-length cDNA and to the use of vaccines and diagnostic assays generated with and derived from the process.

WO 2007/025431 describes a method for producing a recombinant attenuated Newcastle Disease La Sota strain and its use in the preparation of vaccine for the prevention of the diseases caused by Newcastle Disease virus (NDV).

WO 2000/067786 concerns cDNAs for making attenuated, infectious Newcastle disease virus (NDV). Within the scope of the invention are vaccines comprising attenuated, infectious NDV.

**Recombinant paramyxoviruses**

EP-A-0 702 085 relates to genetically manipulated infectious replicating non-segmented negative-stranded RNA virus mutants, comprising an insertion and/or deletion in an open reading frame, a pseudogen region or an intergenic region of the virus genome.

WO 99/66045 relates to genetically modified NDV viruses obtained from full-length cDNA molecules of the virus genome.

WO 00/62735 relates to a method of tumor treatment comprising administering an interferon-sensitive, replication-competent clonal RNA virus, e.g. NDV.

In WO 01/20989 (PCT/US00/26116) a method for treating patients having tumor with recombinant oncolytic paramyxoviruses is described. The tumor is reduced by administering a replication-competent Paramyxoviridae virus. Various methods are described that can be used to engineer the virus genome in order to improve the oncolytic properties.

WO 03/005964 relates to recombinant VSV comprising a nucleic acid encoding a cytokine.

US 2004/0170607 relates to the treatment of melanoma by administering a virus which is not a common human pathogen.

**Genetic manipulation of NDV**

NDV can be genetically manipulated using the reverse genetics technology as described e.g. in EP-A-0 702 085. For example, it is known to make recombinant NDV constructs comprising additional nucleic acids coding for secreted alkaline phosphatase (Zhao and Peeters, 2003), green fluorescent protein (Engel-Herbert et al., 2003), VP2 protein of infectious bursal disease virus (Huang et al., 2004), influenza virus hemagglutinin (Nakaya et al., 2001) and chloramphenicol acetyl transferase (Huang et al., 2001) (Krishnamurthy et al., 2000). None of these recombinant NDV has been constructed for use in the treatment of human disease. The recombinant NDVs were made to study either basic virology of NDV or to develop vaccine strains for poultry. As parental virus strains served lentogenic strains of NDV. These strains do not have significant oncolytic properties.

**Genetic manipulation of viruses**

Methods for genetically manipulating RNA viruses are well known as stated above. Further, genetic manipulation of oncolytic viruses is reviewed e.g. in Bell et al. (2002). RNA viruses as virotherapy agents are reviewed in Russell (2002). The content of any of these documents is herein incorporated by reference.

**Avian Cytokines**

Avian cytokines are reviewed in Staeheli et al. (2001). A genomic analysis of chicken cytokines and chemokines was described in Kaiser et al. (2005).

**Summary of the Invention**

Oncolytic NDV strains have been studied since the early 1960s as tumor therapeutics. Most virus strains used for oncolytic tumor therapy are belonging to the class of mesogenic viruses, that are described as pathogens for poultry. To develop an oncolytic NDV as an antitumoral biological drug, potential existing environmental toxicity, especially the pathogenicity for poultry should be reduced. However NDVs attenuated for poultry must have the continuing ability to lyse tumor cells and keep its oncolytic potential. Existing strategies for the development of vaccines can not be applied, because they are focussing on the stimulation of the bird immune systeme either by application of completely inactivated virus particles or apathogenic lentogenic virus strains. Both vaccine types are no more able to replicate in cancer cells and subsequently have lost the potential to lyse tumor cells.

In the present invention, the attenuation of NDV for poultry without diminishing the oncolytic activity of the virus can be reached with the help of the reverse genetic technology. Recombinant attenuated NDVs are created by the insertion of transgenes coding for cytokines, in particular antiviral or immune-stimulating cytokines.

Furthermore a NDV vaccine is not suitable for such an approach because the objective of a vaccine is to induce a strong immune response with a long lasting immunity to protect the animal against a secondary infection. In the present invention the described attenuation of NDV has the main goal to allow an animal to control the (undesired) primary infection and thus to decrease environmental toxicity and to increase safety of the virus when applied in a therapy of a proliferative disease.

A subject of the present invention is the lysis of tumor cells by an oncolytic virus. An indication for such an oncolytic virus approach is cancer therapy in humans. In contrast a NDV vaccine for poultry as disclosed in US 2003/0224017 is applied to animals and therefore located in the field of animal health care.

Thus, an object of the present invention is a recombinant oncolytic RNA Newcastle Disease Virus comprising at least one transgene coding for an avian cytokine, wherein the recombinant oncolytic RNA Newcastle Disease Virus is obtainable from a velogenic or mesogenic oncolytic RNA Newcastle Disease Virus.

The virus of the present invention may comprise at least one further transgene having therapeutic activity when expressed by a virus-infected tumor cell.

The at least one further transgene is partially allogene or syngene for the host.

In another aspect of the present invention, the virus of the present invention may be an attenuated virus.

"Attenuation" or "attenuated" includes reduction of the pathogenicity of the virus for an avian species and reduction of pathogenicity in a biological assay system that is predictive for avian species (see Example 5).

A further aspect of the present invention is a recombinant oncolytic RNA virus attenuated for poultry comprising a nucleic acid comprising at least one transgene coding for a cytokine. In particular, the virus is attenuated for chicken. The "transgene coding for a cytokine" is in particular the "a transgene coding for an avian cytokine" as described herein.

Further, the invention relates to the nucleocapsid of the recombinant virus of the present invention comprising viral RNA complexed with capsid proteins. Further, the invention relates to an RNA which is RNA of the virus of the present invention. The invention also relates to an RNA complementary to the RNA of the virus of the present invention.

Furthermore, the invention relates to a DNA, e.g. a cDNA encoding the RNA of the present invention and/or a DNA complementary to the RNA of the present invention. Furthermore, the invention relates to the prevention or treatment of tumor diseases, cancer or/and proliferative diseases.

The RNA or/and the DNA of the present invention may be provided in an isolated form.

In the present invention, the cytokine encoded by the transgene as described herein may be secreted from a bird cell infected by the virus of the present invention and, after binding to its appropriate interferon-receptor on the neighbouring cell, may induce an antiviral state in the receptor-carrying cell. Therefore replication of the virus of the present invention in the interferon stimulated bird cell can be inhibited at least partially. The similarity between chicken type I interferons and mammalian type I interferons is very low (<25% identity at amino acid level) (Staeheli et al. 2001).

For this reason a cytokine exhibiting such antiviral effect in a bird cell does not show essentially biological activity in a human cell and may have essentially no influence on virus replication in human tumor cells. Furthermore, essentially no adverse effects by these cytokines are expected in the human organism.

The expression of the transgene encoding a cytokine as described herein leads to a reduced pathogenicity of the virus for an avian species, especially poultry, especially chicken and thereby to a diminished environmental toxicity. The activity of the transgene encoding a cytokine as described herein has essentially no detrimental effect on the therapeutic effect of the virus.

**Detailed description of Preferred Embodiments:**

**Viruses**

The invention generally relates to RNA viruses, preferably negative strand RNA viruses, more preferably such viruses that have both oncolytic properties and can be genetically engineered. Such viruses are:

- paramyxoviruses, preferably Newcastle Disease Virus (NDV), measles virus, mumps virus, Sendai virus;
- orthomyxoviruses, preferably influenza virus;
- rhabdoviruses, preferably vesicular stomatitis virus.

It is preferred that the virus of the present invention is an avian pathogen, in particular a pathogen of poultry, more particular a pathogen of chicken.

It is also preferred that the virus of the present invention is a negative strand RNA virus. The recombinant RNA virus of the present invention may be a paramyxovirus, preferably a Newcastle Disease Virus (NDV). The NDV may be a mesogenic or velogenic strain. It is preferred that the NDV is a mesogenic NDV.

The virus of the present inventon is obtainable from a velogenic or mesogenic oncolytic RNA Newcastle Disease Virus, in particular from strain MTH68.

The virus of the present invention is preferably replication competent.

The virus of the present invention may have a pathogenicity reduced for an avian species, in particular with respect to the virus from which the recombinant virus is obtainable.

It is preferred that in the virus of the present invention, the pathogenicity reduction is a capability of the virus to reduce bird cell lysis about 48h after infection with MOI 0,01 measured by increasing cell viability, whereby cell viability is increased to at least about 25% (such as at least about 25% up to about 50%) surviving cells, more preferably to at least about 50% (such as at least about 50 % up to about 75%) surviving cells and most preferably to at least about 75% (such as at least about 75% up to 100%) surviving cells with respect to the virus from which the recombinant virus is obtainable.

It is also preferred that in the virus of the present invention, the pathogenicity reduction is a survival time prolongation of virus infected chicken embryos in 11 day old embryonated eggs measured by mean death time (MDT) determination, whereby the MDT is prolonged by at least about 15h (such as at least 15 h up to about 20h), more preferably at least about 20h (such as at least 20 h up to about 30h and most preferably by more than 30h compared to the virus from which the recombinant virus is obtainable.

In yet another embodiment, the oncolytic activity of the virus of the present invention for human tumor cells is essentially not reduced.

It is preferred that in the virus of the present invention, the oncolytic activity of the virus for human tumor cells measured by cell viability after 48h after infection is not reduced to more than 50% compared to the virus from which the recombinant virus is obtainable and more preferably is essentially not reduced with respect to the virus from which the recombinant virus is obtainable.

**Cytokines**

The cytokine encoded by the at least one transgene as indicated herein may be any cytokine. In particular, the cytokine may be a cytokine capable of inhibiting at least partially virus replication in a bird cell, in particular in poultry cell, more particular in chicken cell. It is preferred that virus replication is essentially completely inhibited.

The cytokine may be a cytokine having essentially no biological activity in a mammal, in particular in a human being. In this context, "biological activity" refers in particular to the ability of a cytokine to inhibit at least partially virus replication, in particular replication of a virus of the present invention. "Biological activity" also refers to any other activity a cytokine may exhibit in a non-mammalian cell or/and in a non-mammal.

The cytokine may be a cytokine capable of inhibiting at least partially virus replication in a bird, in particular in poultry, more particular in chicken, and may have essentially no biological activity in a mammal, in particular in a human being.

The cytokine may be selected from avian cytokines, in particular from poultry cytokines, more particular from chicken cytokines.

The cytokine may be selected from interferons, in particular from avian interferons, more particular from chicken interferons, more particular from chicken type I interferons.

The interferon may be interferon-beta or a member of the interferon-alpha family.

In a preferred embodiment, the recombinant oncolytic RNA virus is an NDV comprising a nucleic acid comprising a transgene encoding a chicken interferon-alpha or/and chicken interferon-beta.

**Transgenes**

A transgene is defined in the context of the NDV genome as additional nucleic acids that are introduced in the viral genome. The nucleic acids can be selected from different genomic sources (e.g. NDV genome, different virus class, prokaryotic or eukaryotic sources, mammalian or non-mammalian species). Also fused transgenes from two or more different genomic sources are possible. Also synthetic transgenes based on de novo synthesis of nucleic acid sequences can be constructed. The nucleic acids must be located within at least one transcriptional cassette. Preferably the transgene is translated into a protein in the infected cell. For this reason the transgenic sequence should include a translational start and stop-codon.

The recombinant oncolytic virus of the present invention may comprise a nucleic acid encoding at least one further transgene independently selected from transgenes coding for binding proteins, prodrug-converting enzymes, and proteases.

The at least one further transgene codes preferably for a binding protein that has a therapeutic activity when expressed by the virus-infected tumor cell.

In another preferred embodiment, the at least one further transgene codes for a prodrug-converting enzyme that has a therapeutic activity when expressed by the virus-infected tumor cell.

In yet another preferred embodiment, the at least one further transgene codes for a protease that has a therapeutic activity when expressed by the virus-infected tumor cell.

If not indicated otherwise, "transgene" or "at least one transgene" as used herein refers to a transgene encoding an avian cytokine, or the at least one further transgene having therapeutic activity when expressed by a virus-infected tumor cell, or a combination thereof. This definition of "transgene" applies in particular in the position and number of transgenes within the virus genome, as described herein, if not indicated otherwise.

The RNA virus, the genome, antigenome, nucleocapsid and/or DNA molecule of the present invention may comprise the transgene as described herein which may be located within the transcriptional cassette as described herein.

The recombinant oncolytic virus of the present invention may comprise at least two, at least three, at least four, or at least five nucleic acids each comprising a transgene as described herein. The recombinant oncolytic virus of the present invention may comprise at the maximum five nucleic acids each comprising a transgene as described herein. In particular, recombinant oncolytic virus of the present invention comprises one, two, three, four, or five nucleic acids each comprising a transgene as described herein. If the recombinant virus of the present invention comprises at least two transgenes, they may be identical or different.

The nucleic acid comprising the at least one transgene may be located at any position between the reading frames of the viral genes. In particular, the nucleic acid comprising the at least one transgene may be located 5' of the N gene, between the N and the P gene, between the P and the M gene, between the M and the F gene, between the F and the HN gene, between the HN and the L gene, or/and 3' of the L gene. It is preferred that the nucleic acid is located in a more 5' position, such as 5' of the N gene, between the N and the P gene, or/and between the P and the M gene, as such location leads to an improved expression compared with a more 3' location.

Preferably the virus of the present invention exhibits a tumor-selective infection that leads to a tumor-selective expression of the transgene as described herein.

The recombinant RNA virus of the present invention may comprise in total up to five transgenes, up to four transgenes, or up to three transgenes.

In the present invention, the transgene encoding an avian cytokine, or the at least one further transgene having therapeutic activity when expressed by a virus-infected tumor cell, or a combination thereof is preferably heterologous to the oncolytic RNA virus on which the recombinant RNA virus of the present invention is based. The term "heterologous" as used herein refers to the complete gene or a part thereof, which may be the coding region of the gene or a part thereof.

The heterologous nucleic acid may be an artificial nucleic acid or may be obtained from natural sources or by recombination of at least two nucleic acids selected from nucleic acids obtained from natural sources and/or artificial nucleic acids. "Natural sources" include animals such as mammals, plants, fungi, and microorganisms such as bacteria, protozoa and viruses, which may be different from oncolytic RNA viruses of the present invention.

The transgene may also encode for a fusion protein.

In another embodiment of the present invention, the transgene encoding an avian cytokine and the at least one further transgene having therapeutic activity when expressed by a virus-infected tumor cell may be located on at least two separated transcription units.

At least one transcription unit comprising the nucleic acid comprising the at least one transgene encoding an avian cytokine may also be transcribed in a tumor cell as described herein.

At least one transcription unit comprising the nucleic acid comprising the at least one second transgene having therapeutic activity when expressed by a virus-infected tumor may also be transcribed in a bird cell as described herein.

At least two separated transcription units each may be transcribed in a tumor cell as described herein and in a bird cell as described herein.

In an alternative preferred embodiment, at least one transgene encoding an avian cytokine and the at least one further transgene having therapeutic activity when expressed by a virus-infected tumor cell are translated in a tumor cell as described herein and in a bird cell as described herein.

**Binding proteins**

In the oncolytic recombinant RNA virus of the present invention, the at least one further transgene having therapeutic activity when expressed by a virus-infected tumor cell may code for a binding protein.

Therefore subject of the present invention is a pharmaceutical composition comprising a recombinant oncolytic virus of the present invention, a virus genome of the present invention, a virus antigenome of the present invention, and/or a DNA molecule of the present invention as an active ingredient optionally together with pharmaceutically acceptable carriers, diluents and/or adjuvants, which virus, virus genome, antigenome and/or DNA molecule comprises at least one one further transgene having therapeutic activity when expressed by a virus-infected tumor cell encoding for a binding protein.

Binding proteins are proteins, which, when expressed in a target cell, are capable of binding to a component of said cell and/or a neighbouring cell. Preferably, binding proteins are proteins which bind to intracellular components.

In a preferred embodiment, a binding protein is selected from the following group consisting of a natural ligand, a genetically modified ligand, a recombinant soluble domain of a natural receptor and a modified version thereof, a peptide ligand, a polypeptide ligand, an antibody molecule and fragments and derivatives thereof, and an antibody-like molecule like an ankyrin-repeat protein and fragments and derivatives thereof.

An incomplete review of high-affinity binding frameworks is given by Ladner and Ley (2001).

The binding proteins as described herein might be of human, murine or closely related origin or a chimeric version, i.e. a protein which may be a fusion protein comprising sequences from different species, e.g. human and mouse.

The recombinant binding molecules based on the description above can be monomeric, dimeric, trimeric, tetrameric or multimeric. The recombinant binding molecules based on the description above can be monospecific, bispecific or multispecific.

The preferred binding proteins are selected from binding proteins having a therapeutic activity.

A natural ligand as described herein can be a growth factor or a peptide. A genetically modified ligand may be an analogue of a naturally occurring growth factor or peptide.

Recombinant soluble domains of a natural receptor or modified versions of it as described herein are recombinantly expressed soluble extracellular domains of a cell-surface receptor and/or fragments of it, a recombinantly expressed soluble extracellular domain of a cell adhesion molecule and/or fragments thereof.

Antibody molecules as mentioned above may be monoclonal immunoglobulin antibodies of any known specificity and isotype, fragments thereof and/or fragments thereof fused to effector proteins. The antibody molecules may be chimeric, humanized or human antibodies. Antibody fragments contain at least one antigen-binding domain of an antibody. Antibody fragments have been described extensively in the literature (reviewed eg. in Allen (2002), herein incorporated by reference). Preferred examples are single-chain Fv fragments, Fab fragments, F(ab2'), domain-deleted versions called minibodies, and other immunoactive portions, fragments, segments and other smaller or larger partial antibody structures wherein the latter possess sufficient targeting properties or immunological stimulatory or inhibitory activity so as to be therapeutically useful within the methods of the present invention.

Such antibodies may be derived from hybridoma cloning experiments by use of transgenic mice or from phage display selections, ribosome display selections, or colony filter screening of antibody libraries containing human antibody sequences or related methodologies.

Binding proteins with antibody like properties as described herein may be genetically modified proteins or domains of it in which one or more peptide loops are randomized on the level of amino acids in such a way that high affinity binding molecules with high specificity can be enriched against any antigen from libraries of such molecules by phage display, ribosome display, colony filter screen or related methodologies. The selected proteins usually have high thermal and thermodynamic stability and are well expressed in recombinant expression systems such as E. coli, yeast, insect and mammalian expression system. Examples for such binding proteins with antibody like properties are ankyrin repeat proteins as described in Binz et al. (2004), the lipocalins as described in Skerra (2000), the gamma-crystallins as described in DE 199 32 688.6, the modified protein A scaffold (affibodies) as described in Hogbom et al. (2003), or Nord et al. (2000) or the fibronectin framework and others. Antibody-like molecules can be monomeric or repetitive molecules either constructed as single-chain molecules or as multichain molecules wherein the antibody-like molecule possesses sufficient targeting properties or immunological stimulatory or inhibitory activity so as to be therapeutically useful within the methods of the present invention.

Another subject of the present invention is a method for treatment of a proliferative disease, in particular a hyperproliferative disease, such as a tumor or cancer, comprising administering in a pharmaceutically effective amount to a subject in need thereof a recombinant oncolytic virus of the present invention, a virus genome of the present invention, a virus antigenome of the present invention, and/or a DNA molecule of the present invention comprising at least one further transgene having therapeutic activity when expressed by a virus-infected tumor cell encoding for a binding protein as described herein.

**Binding molecules with additional function**

The binding protein may be a fusion protein comprising at least one binding domain, e.g. from an antibody, and at least one heterologous domain. "Heterologous" has the meaning as discussed above in the context of heterologous genes.

The binding proteins described above are able to deliver a payload to a disease specific site (e.g. a tumor) as a so called intrabody or as extracellular available binding protein. The delivered payload can be a heterologous domain, e.g. a toxin such as human RNAse (De Lorenzo et al., 2004) (Zewe et al., 1997) Pseudomonas exotoxin (Chaudhary et al., 1989) (Kreitman and Pastan, 1995) (Batra et al., 1992) , Diphtheria toxin (Kreitman et al., 1993) (Chaudhary et al., 1990) (Batra et al., 1991), or an enzyme such as beta-galactosidase, beta-glucuronidase (Roffler et al., 1991) (Wang et al., 1992) (Bosslet et al., 1992), beta-glucosidase (Rowlinson-Busza, 1992), carboxypeptidase, (Antoniw et al., 1990), (Bagshawe et al., 1988), beta-lactamase with therapeutic efficacy, or an immune-stimulatory protein with cytokine activity such as IL-2, iL-12, TNF-alpha, IFN-beta or GM-CSF (see e. g. review by Allen (2002).

In another example the binding proteins described above have themselves antagonistic or agonistic efficacy which is therapeutically useful. Examples for antagonistic/blocking binding molecules are the VEGF inhibitory antibody Avastin (Ferrara et al., 2004), the HER2/neu receptor blocking antibody Herceptin (Noonberg and Benz, 2000) or the EGF-receptor blocking antibody Erbitux (Herbst and Langer, 2002). Agonistic binding proteins can be binding proteins which induce for example apoptosis (Georgakis et al., 2005) or have regulatory activity on DNA, RNA or proteins (e.g. induce transcription, stabilize proteins). The review by (Adams and Weiner, 2005) describes various therapeutic antibodies that could also be incorporated into an oncolytic virus

**Prodrug-converting enzymes**

In the oncolytic recombinant RNA virus of the present invention, the at least one further transgene having therapeutic activity when expressed by a virus-infected tumor cell may code for a prodrug-converting enzyme.

A prodrug is a derivative or a precursor of a therapeutically active compound, which can be enzymatically converted into the active compound. Prodrug-converting enzymes are enzymes capable of converting a prodrug into the therapeutically active drug.

Therefore subject of the present invention is a pharmaceutical composition comprising a recombinant oncolytic virus of the present invention, a virus genome of the present invention, a virus antigenome of the present invention, and/or a DNA molecule of the present invention as an active ingredient optionally together with pharmaceutically acceptable carriers, diluents and/or adjuvants, which virus, virus genome, antigenome and/or DNA molecule comprises at least one one further transgene having therapeutic activity when expressed by a virus-infected tumor cell encoding for a prodrug-converting enzyme.

The pharmaceutical composition may further comprise a prodrug which can be converted into a therapeutically active compound by the prodrug-converting enzyme encoded by the virus, virus genome, antigenome and/or DNA molecule. The pharmaceutical composition may be suitable for treatment and/or alleviation of a proliferative disorder.

The prodrug may be formulated in a single composition with the recombinant oncolytic virus of the present invention, a virus genome of the present invention, a virus antigenome of the present invention, and/or a DNA molecule of the present invention as an active ingredient, or may be formulated in a composition distinct from the oncolytic virus formulation.

If the oncolytic recombinant RNA virus of the present invention encodes for a prodrug-converting enzyme, the oncolytic virus of the present invention causes selective expression of the prodrug-converting enzyme in a virus-infected target cell (in particular a tumor cell) which is usually not or not sufficiently expressing the prodrug converting enzyme. Thus, during treatment of a subject in need thereof, the prodrug is specifically converted into the pharmaceutical active compound in a target cell, in particular in a tumor cell, but may essentially not be converted into the therapeutically active compound in a non-target cell, in particular in a healthy cell of the subject to be treated. Thus, undesired side-effect of the therapeutically active compound are reduced compared with treatment of the therapeutically active compound alone.

In the context of the present invention, the prodrug may be a derivative or a precursor of a therapeutically active compound suitable for treatment and/or alleviation of a proliferative disorder, tumor or/and cancer, which prodrug can be converted by a prodrug converting enzyme. The prodrug may be a compound known by a person skilled in the art. Derivatives and/or precursors are known by a person skilled in the art.

It is preferred that the prodrug is essentially pharmaceutically inactive and/or nontoxic.

Examples of prodrug-converting enzymes of the present invention are beta-glucuronidase, beta-galactosidase, beta-glucosidase, carboxypeptidase, beta-lactamase, D-amino acic oxidase. Further examples are known by a person skilled in the art.

It is preferred that the prodrug-converting enzyme is essentially not expressed in non-tumor cells.

The prodrug-converting enzyme may be obtained from an organism selected from mammals, plants, fungi, and microorganisms such as bacteria, protozoa and viruses.

A most preferred combination of the prodrug-converting enzyme and a prodrug is E. coli beta-glucuronidase and a prodrug which can be converted by beta-glucuronidase into an active cytotoxic compound. An example is HMR1826 (doxorubicin-glucuronide) which can be converted into doxorubicin which is a known compound for treatment of cancer.

Another subject of the present invention is a method for treatment of a proliferative disease, in particular a hyperproliferative disease, such as a tumor or cancer, comprising administering in a pharmaceutically effective amount to a subject in need thereof a recombinant oncolytic virus of the present invention, a virus genome of the present invention, a virus antigenome of the present invention, and/or a DNA molecule of the present invention comprising at least one further transgene encoding for a prodrug converting enzyme as described herein.

In particular, subject of the present invention is a method for treatment of a proliferative disease, in particular a hyperproliferative disease, such a cancer, comprising administering in a pharmaceutically effective amount to a subject in need thereof

> (i) a recombinant oncolytic virus of the present invention, a virus genome of the present invention, a virus antigenome of the present invention, and/or a DNA molecule of the present invention comprising at least one transgene encoding for a prodrug-converting enzyme, and
> (ii) a prodrug suitable for treatment of the proliferative disease, which prodrug can be converted into a pharmaceutically active compound by the prodrug-converting enzyme of (i).

The method may comprise the administration of a single pharmaceutical composition comprising both components (i) and (ii), or may comprise the administration of two distinct pharmaceutical compositions, one of which comprises component (i) and the other comprises (ii).

### Proteases

In the oncolytic recombinant RNA virus of the present invention, at least one further transgene having therapeutic activity when expressed by a virus-infected tumor cell may code for a protease.

Therefore subject of the present invention is a pharmaceutical composition comprising a recombinant oncolytic virus of the present invention, a virus genome of the present invention, a virus antigenome of the present invention, and/or a DNA molecule of the present invention as an active ingredient optionally together with pharmaceutically acceptable carriers, diluents and/or adjuvants, which virus, virus genome, antigenome and/or DNA molecule comprises at least one further transgene having therapeutic activity when expressed by a virus-infected tumor cell encoding for a protease. The pharmaceutical composition may be suitable for treatment and/or alleviation of a proliferative disorder.

If the oncolytic recombinant RNA virus of the present invention encodes for a protease, the oncolytic virus of the present invention may cause selective expression of the protease in a virus-infected target cell (in particular a tumor cell) which is usually not or not sufficiently expressing the protease. Thus, during treatment of a subject in need thereof, the protease may irreversibly cleave a target polypeptide in a target cell, thereby inhibiting proliferation and/or growth of the target cell or killing the target cell, but may essentially not cleave the target molecule in a non-target cell, in particular in a healthy cell of the subject to be treated. By this strategy, undesired side-effects of protease treatment are reduced.

It is preferred that the protease is a sequence-specific protease. More preferred is a protease specifically cleaving a target polypeptide. The protease may either be of natural origin and may be derived from any species or it may be engineered. Amino acid sequences suitable for a specific cleavage of a predetermined target polypeptide can be determined by a person skilled in the art, e.g. on the basis of publicly available sequence databases. US 2005-0175581 and US 2004-0072276 describe the generation of protein-engineered proteases with a predetermined substrate specifity.

These two documents are herein included by reference.

The target molecule of the protease may be any target molecule as described below for targets of binding proteins.

Another subject of the present invention is a method for treatment of a proliferative disease, in particular a hyperproliferative disease, such as a tumor or cancer, comprising administering in a pharmaceutically effective amount to a subject in need thereof a recombinant oncolytic virus of the present invention, a virus genome of the present invention, a virus antigenome of the present invention, and/or a DNA molecule of the present invention comprising at least one further transgene having therapeutic activity when expressed by a virus-infected tumor cell encoding for a protease.

The transgene of the present invention may encode a fusion protein of a prodrug-converting enzyme as defined above, a binding molecule as defined above and/or a protease as defined above. Especially preferred is a fusion protein of a prodrug-converting enzyme and a binding molecule or a fusion protein of a protease and a binding molecule.

**Therapeutic Applications**

The present invention relates to a pharmaceutical composition which comprises as an active ingredient a virus as described herein, a nucleocapsid of the virus, a genome of the virus or a DNA molecule encoding the genome or/and an antigenome of the virus, optionally together with pharmaceutically acceptable carriers, diluents and/or adjuvants.

The pharmaceutical composition may be provided as a solution, suspension, a lyophilisate or in any other suitable form. In addition to the active ingredient, the composition may comprise carriers, buffers, surfactants and/or adjuvants as known in the art. The composition may be administered e.g. orally, topically, nasally, pulmonally or by injection locally or intravenously. The pharmaceutical composition is administered in a pharmaceutically effective amount depending on the type of disorder, the patient's condition and weight, the route of administration etc. Preferably $10^9$ to $10^{12}$ virus particles, $10^8$ to $10^{11}$, $10^7$ to $10^{10}$, or $10^6$ to $10^9$ virus particles are administered per application. The oncolytic therapy may be optionally combined with other tumor therapies such as surgery, radiation and/or chemotherapy such as cyclophosphamide treatment and/or hyperthermia treatment.

Yet another aspect is a method for treatment of a proliferative disease or/and cancer, comprising administering in a pharmaceutically effective amount to a subject in need thereof a recombinant oncolytic virus comprises as described herein, a nucleocapsid of the virus, a genome of the virus or a DNA molecule encoding the genome or/and an antigenome of the virus, optionally together with pharmaceutically acceptable carriers, diluents and/or adjuvants.

According to the present invention, a recombinant oncolytic paramyxovirus can express a soluble binding protein, a prodrug-converting enzyme and/or a protease that may remain either in the infected cell or may be secreted, such as an antibody, an antibody fragment, an ankyrin repeat protein or another binding molecule as specified below.

As an example NDV, the strain MTH68 was chosen in the present application because it has an inherent oncolytic property with promising data from experimental clinical treatments of patients (Sinkovics and Horvath, 2000). In principle, however, most NDV strains with multibasic fusion protein cleavage sites may be used as oncolytic agents for the treatment of tumors. The reverse genetics technology is applicable to all strains.

Binding proteins as described herein have been demonstrated to be of high therapeutic potential.

The combination of oncolytic NDV with therapeutic binding proteins, prodrug-converting enzymes and/or proteases of the above described properties will have additional or even synergistic efficacy of two therapeutical principles. The oncolytic self-replicating virus targets the binding protein drug, the prodrug-converting enzyme and/or the protease to the preferred site of action where it is expressed in situ in high local concentrations. Such protein expression is expected to be very selective and the binding protein, the prodrug-converting enzyme and/or the protease with its respective mode of action will add to the intrinsic therapeutic oncolytic activity of the NDV. Based on the replication competent nature of the used virus and the selective replication in tumor cells the amount of expressed transgene [binding protein, the prodrug-converting enzyme and/or protease] is expected to be roughly proportional to the mass of the tumor.

Antibody molecules or antibody like molecules or derivatives thereof are ideal binding proteins to be used with the NDV-system. Antibody molecules have been the subject of intensive research and technologies are now available to generate antibody molecules which are non-immunogenic, very selective and of high affinity. The local expression of antibody molecules at high concentrations lead to very significant agonistic or antagonistic efficacy or efficient targeting of effector molecules with reduced toxicity profile compared to standard therapy.

The use of antibody-like molecules in the NDV system is expected to be even superior. These molecules are designed for selective high affinity binding with very high thermal stability and yield compared to normal antibodies. In the case of the ankyrin-based antibody-like molecules the repetitive nature of the molecule can be finetuned according to the respective target for optimized targeting, binding, inhibition or activation. Also different binding specificities can be combined within one ankyrin molecule, exploiting the possibility of joining in one ankyrin-repeat molecule several units with different binding specificities. This modular structure allows the multivalent binding of greater protein surfaces than it is possible for antibodies, which can be extremely important in blocking protein-protein interactions. The modular structure can also be exploited to block several effectors with only one single blocking ankyrin-repeat-protein.

Since the ankyrin-repeat-molecules are extremely stable even under reducing condition these molecules can be designed to target proteins inside the cell ("Intrabody").

Also possible is the use of libraries of binding protein-coding sequences with NDV for in vivo target identification.

Possible targets for binding molecules or/and proteases can be all structures of a target cell or of the extracellular matrix surrounding the target cell which can be recognized by the described binding proteins or/and proteases and which are relevant to a certain type of pathological phenotype. These can be structural proteins, enzymes, growth factors, growth factor receptors, integrins, transcription factors etc.

Even targets that are not drugable by small molecules (protein-protein interactions, DNA-binding etc.) can be addressed by this invention.

The combination of the oncolytic NDV and therapeutic binding proteins, prodrug-converting enzymes and/or proteases as described herein are envisaged for the treatment of inflammatory disease e.g. rheumatoid arthritis and of cancer.

For the treatment of cancer all pathways which contribute to the development of cancer can be targeted. These pathways are: self-sufficiency in growth signals, insensitivity to growth-inhibitory (antigrowth) signals, evasion of apoptosis, limitless replicative potential, sustained angiogenesis, and tissue invasion and metastasis. A summary of these pathways is given in (Hanahan and Weinberg, 2000). Signaling pathways that are involved in the tumorigenesis process and can be targeted by the described approach are the receptor tyrosine kinase pathway (RTK) pathway, RB and p53 pathway, apoptosis pathway, APC pathway, HIF1 pathway, GLI pathway, PI3K pathway and the SMAD pathway. A detailed description of these signaling pathways are given in Vogelstein and Kinzler (2004).

Other signaling pathways where described binding proteins could interfere with are the ras, Wnt and Hedgehog pathway, where for example protein protein interactions can be blocked.

Examples of binding proteins intervening beneficially in the above described pathways in cancer cells are:

- blocking proteins of autonomous active growth factor receptors (eg. EGFR, Met)
- competitive binders for growth factors (antagonists)
- blocking proteins for Rb-phosphorylation
- blocking proteins for E2F-dependent transcription
- stabilizers for p53
- antagonistic binders for antiapoptotic proteins (e.g. Bcl-2)
- antagonistic binders for cyclins
- antagonistic binders for Ras effectors (eg. GEFs)
- antagonistic binders for hypoxia induced proteins (e.g. HIF1$\alpha$)
- inhibitors of transcription factors that interfere with dimerization, DNA-binding or/and cofactor binding (eg. Myc/Max)
- inducers of differentiation
- inhibitors of smad signalling/translocation
- inhibitors of cellular adhesion interactions (cadherins, integrins, eg. $\alpha5\beta1$, $\alpha v\beta3$)
- inhibitors of enzymes that degrade the extracellular matrix (eg. MMPs)
- antagonistic binders for proangiogenic ligands (eg. soluble VEGF-R)
- inhibitors of mitotic kinases (eg. Plk-1)
- antagonistic binders to proangiogenic receptors
- inhibitors of scaffold complex formation (eg. KSR/Ras)
- inhibitors of translation initiation (eIF4E, EIF2a)

The protease of the present invention, the prodrug-converting enzyme and/or the therapeutically active compounds derived from prodrugs of the present invention by the prodrug-converting enzyme may also beneficially intervene in the above described pathways of cancer cells.

**Recombinant virus**

Recombinant virus means a virus that has an engineered defined alteration in its genomic RNA sequence. This alteration may be one or more insertions, deletions, point mutations or combinations thereof.

A recombinant RNA virus of the present invention may comprise the full genomic sequence of a natural (unmodified) RNA virus or a sequence derived thereof and may additionally comprise at least one recombinant transcriptional cassette. The at least one transcriptional cassette may be located in between two genes (transcriptional units) of the viral genome. In this case, the at least one transcriptional cassette is flanked by transcriptional start and stop sequences. The at least one transcriptional cassette may also be located within a transcriptional unit of the viral genome. In this case, no additional transcriptional start and stop sequences are required.

The at least one transcriptional cassette may comprise restriction sites, such as PacI or/and AscI, which may be unique. If two transcriptional cassettes are present, they may comprise different restriction sites.

It is preferred that the RNA virus of the present invention comprises one or two recombinant transcriptional cassettes.

In the at least one transcriptional cassette of the present invention, there is a transgene located, which may encode for a binding protein,a prodrug-converting enzyme and/or a protease as described herein.

Any intergenic region between each of two genes (transcriptional units) of the viral genome is suitable for introducing the at least one recombinant transcriptional cassette. If more than one recombinant transcriptional cassette is present, they may be located in the same or different intergenic regions. It is preferred that at least one recombinant transcriptional cassette is located between the viral F and HN genes, in particular if the RNA virus of the present invention is a recombinant Newcastle Disease Virus.

There is no known upper limit for the size of the genome of Paramyxoviridae. Therefore, there is no upper limit for the number and size of transgenes introduced into the recombinant RNA virus of the present invention. It is preferred that the transgene (transgene encoding an avian cytokine, or the at least one further transgene having therapeutic activity when expressed by a virus-infected tumor cell, or a combination thereof, as described herein) independently have a size of up to about 10 kb, more preferred up to about 5 kb, most preferred up to about 2 kb.

In the expression (including transcription of the viral RNA into mRNA and translation of the mRNA) of the transgene as described herein, expression control sequences such as transcriptional start and stop sequences and sequences controlling the translation are used. The expression control sequences of an RNA virus may be used which may be the RNA virus on which the recombinant RNA virus of the present invention is based. In particular, transcriptional start and stop sequences may be obtained from an RNA virus. Expression control sequences may also be obtained from a target cell, in particular sequences controlling the translation and/or protein transport.

Due to the replication mechanism of Paramyxoviridae, the genomic or antigenomic RNAs usually do not appear as naked RNAs. The genomic and antigenomic RNAs are assembled with the nucleoprotein. Therefore, a further subject of the present invention is a nucleocapsid of a recombinant oncolytic RNA virus of the present invention. The nucleocapsid comprises the RNA molecule encoding the genome or/and the antigenome of the RNA virus and the nucleocapsid protein. The nucleocapsid may also comprise the polymerase protein L or/and the phosphoprotein P.

Also subject of the present invention is the anti-genome of the genome of the present invention as described herein.

A further aspect of the present invention is a DNA molecule encoding the genome or/and the anti-genome of a recombinant oncolytic RNA virus of the present invention. The DNA molecule may be a plasmid. The DNA molecule of the present invention can be used for genetically engineering the RNA virus of the present invention. Further, the DNA molecule may be used for producing the RNA virus of the present invention. Therefore, the DNA molecule may be operatively linked to a transcriptional control sequence e.g. a prokaryotic or eukaryotic transcription control sequence.

Another aspect of the present invention is a method for producing a recombinant oncolytic RNA virus expressed from a DNA molecule encoding the genome and/or the anti-genome of a recombinant oncolytic virus of the present invention, in particular the recombinant oncolytic NDV of the present invention.

A further aspect of the present invention is a cell comprising the recombinant oncolytic virus of the present invention, a virus genome of the present invention, a virus anti-genome of the present invention and/or a DNA molecule of the present invention. The cell may be a prokaryotic cell or a eukaryotic cell. The cell may be a cell line, in particular a mammalian cell line, more particularly a human or murine cell line. The cell may be used in the method of the present invention for producing the RNA virus of the present invention. Suitable systems for transcribing a DNA molecule are known by a person skilled in the art, e.g. in prokaryotic systems such as E. coli or eukaryotic systems such as HeLa or CHO.

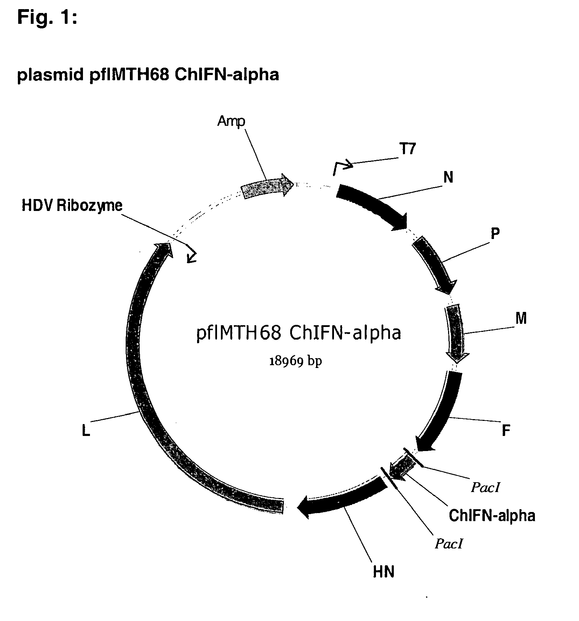

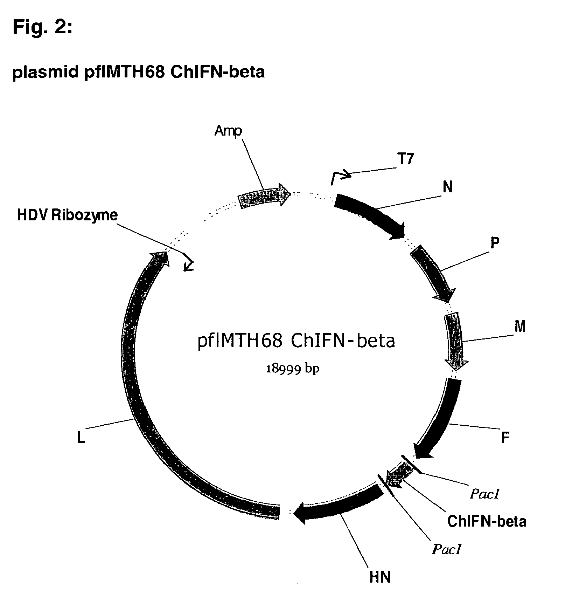

Yet another aspect is an oncolytic RNA virus, a genome or anti-genome thereof or a DNA molecule comprising the full set of genes of Paramyxoviridae or a set of genes of Paramyxoviridae in which at least one gene or intergenic region is genetically modified, and further comprising at least one recombinant transcriptional cassette as described herein. Such a virus, genome or antigenome or DNA molecule may be used for the manufacture of a medicament and/or treatment of cancer. Such RNA virus, genome, anti-genome or DNA molecule is suitable for constructing a recombinant Paramyxoviridae virus, in particular a recombinant Newcastle Disease Virus by genetic engineering techniques in order to introduce a recombinant sequence into the transcription cassette. For this purpose, the at least one transcription cassette may comprise a restriction site. If more than one transcription cassettes are present, the unique restriction sites of the transcriptional cassettes may be different. An example is plasmid pfIMTH68_Asc_Pac of Fig. 1 of WO 2006/050984, the disclosure of which is included herein by reference. Another example is pfIMTH68 murine IgG EDB as disclosed in Fig. 2 of WO 2006/050984, the disclosure of which is included herein by reference.

**Therapeutical relevance**

Treatment of cancer or/and of a tumor includes inhibition of tumor growth, preferably the killing of the tumor cells or the blocking of proliferation in a time gap by infection. NDV replicates selectively in tumor cells.

The virus of the present invention can be used to treat proliferative disorders, in particular hyperproliferative disorders. Preferably neoplasms can be treated with the described virus, preferably cancers from the group consisting of lung, colon, prostate, breast and brain cancer can be treated.

More preferably a solid tumor can be treated.

More preferably a tumor with low proliferation rate can be treated. Examples of tumors with low proliferation rate are prostate cancer or breast cancer.

More preferably a brain tumor can be treated.

More preferably a glioblastoma can be treated.

Mammals include human beings, mice, and rats.

**Manufacture of the recombinant RNA virus**

The recombinant RNA virus of the present invention, in particular the recombinant NDV, can be constructed as described in Romer-Oberdorfer et al. (1999). The construction of the new nucleic acid sequences is on the level of the cDNA which then is translated into RNA within a eucaryotic cell using the following starting plasmids: pCITE P, pCITE N, pCITE L, pX8δT fINDV.

NDV can be any strain of Newcastle Disease Virus, more preferred a strain that is oncolytic in its wildtype form.

The plasmid pX8δT is described in EP 0 702 085 (Conzelmann KK).

The recombinant RNA virus of the present invention, in particular the recombinant NDV, can be recovered initially from T7 polymerase expressing cells, eg. BHK T7 cells or transiently with T7 polymerase transfected CHO cells. It can be amplified in cells like 293, CEC32, HT29 or A431. It can also be amplified in the allantoic fluid of embryonated chicken eggs.

The recombinant RNA virus, in particular the recombinant NDV, is stored under the following conditions. The recombinant RNA-virus, in particular NDV is stable in 5% D-mannitol / 1% (w/v) L-lysine / pH 8,0 or standard cell culture medium.

At -20°C for up to one month.
At -80°C for up to 10 years.

The recombinant RNA virus of the present invention may be manufactured using a wild type-virus or a recombinant virus as starting material. Also a nucleic acid, such a DNA, including such wild-type or recombinant sequence, may be used. For instance, the recombinant RNA virus or/and DNA molecule as described in WO 2006/050984 may be employed as starting material. An example is plasmid pfIMTH68_Asc_Pac of Fig. 1 of WO 2006/050984. Another example is pfIMTH68 murine IgG EDB of Fig. 2 of WO 2006/050984. The diclosure of WO 2006/050984 is included herein by reference. In particular, the disclosure of WO 2006/050984 concering recombinant oncolytic RNA viruses and the construction thereof is included herein by reference.

**Use of the recombinant NDV as a Medicament**

The recombinant RNA virus of the present invention, in particular the purified recombinant NDV according to the invention can be used as a medicament, because it shows pharmacological effects.

The recombinant RNA virus, in particular the NDV of the invention, the virus genome, antigenome, nucleocapsid and/or DNA molecule of the present invention can be used for the manufacture of a medicament especially for prevention, alleviation or/and treatment of cancer, a tumor or/and a proliferative disease, especially for prevention, alleviation or/and treatment of cancer, such as lung cancer, prostate cancer, brain cancer, colon cancer, breast cancer.

The pharmaceutical composition of the present invention optionally comprises pharmaceutically acceptable carrier and diluents. Such carrier and diluents are described in Remington's Pharmaceutical Science, 15th ed. Mack Publishing Company, Easton Pennsylvania (1980). The virus titers used in the pharmaceutical composition or/and applied in the method of treatment of the present invention may be in the range of $10^9$ to $10^{12}$ pfu per dose, in a range of $10^8$ to $10^{11}$ pfu, in a range of $10^7$ to $10^{10}$ pfu or in a range of $10^6$ to $10^9$ pfu dependent on the indication of treatment.

The pharmaceutical composition of the present invention may be used for the prevention or/and treatment of a proliferative disorder, such as cancer.

The pharmaceutical composition of the present invention may comprise an emulsion of the recombinant oncolytic RNA virus of the present invention, in particular the NDV of the invention and may be administered by inhalation, intravenous infusion, subcutaneous injection, intraperitoneal injection or intratumoral injection.

In the method of the present invention for the prevention or/and treatment of a proliferative disorder, in particular cancer, a pharmaceutically effective amount is a titre of the oncolytic RNA virus of the present invention, in particular the NDV of the present invention, the virus genome of the present invention, or the DNA molecule of the present invention which prevents, alleviates or/and suppresses the disease.

For the therapeutic effect the acceptable dosis may depend for example on the construct, the patient, the ways of administration and the type of cancer.

It is preferred that the subject (the patient) is a mammal, more preferably a human patient.

The present invention is further illustrated by the following Figures and Examples.

**Figure legends**

**Figure 1** describes plasmid pflMTH68 ChIFN-alpha comprising the full genome of NDV and one transcription cassette comprising the chicken interferon-alpha (ChIFN-alpha) transgene.

**Figure 2** describes plasmid pflMTH68 ChIFN-beta comprising the full genome of NDV and one transcription cassette comprising the chicken interferon-beta (ChIFN-beta) transgene.

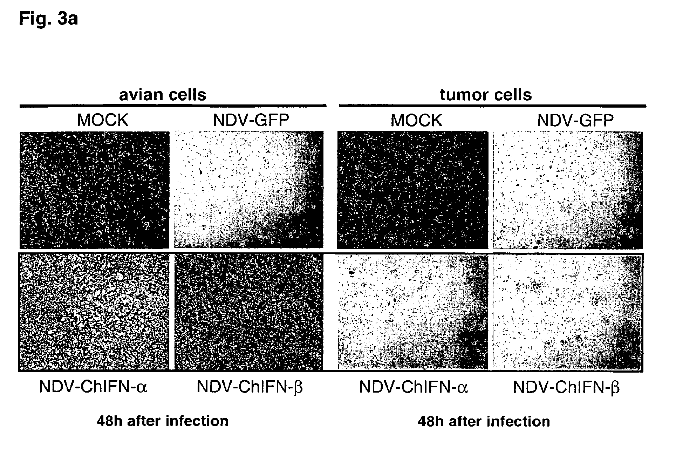

**Figure 3a** demonstrates that the avian CEC-32 cell line is partial protected from lysis 48h after infection with NDV-ChIFN-alpha and strong protected after infection with NDV-ChIFN-beta. Infection with a GFP expressing NDV completely destroys the CEC-32 monolayer. In contrast no difference in the lytic effect between the three viruses NDV-GFP, NDV-ChIFN-alpha and NDV-ChIFN-beta is seen after infection of the tumorigenic Hela cell line. Independend of the used virus the Hela cell monolayer is 48h after infection completely destroyed.

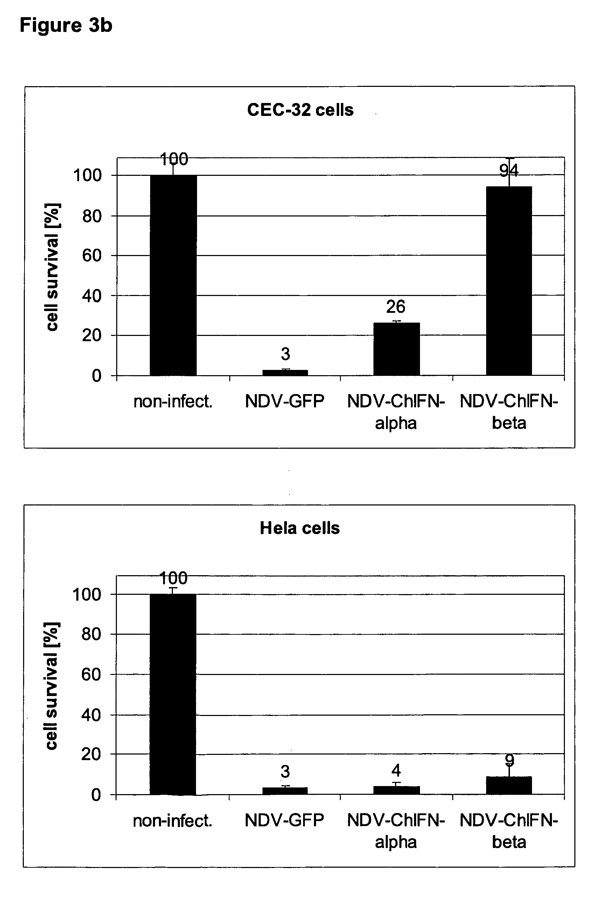

**Figure 3b** shows a quantification of the cell survival of CEC-32 and Hela cells 48h after infection with the viruses NDV-GFP, NDV-ChIFN-alpha and NDV-ChIFN-beta. After infection with NDV-GFP nearly a complete killing of the CEC-32 cells is observed, only 3% of the cells are viable. The infection of the quail cells with the NDV-ChIFN-alpha keeps 26% of the infected cells alive. The best protection is observed after infection with the NDV-ChIFN-beta virus, 96% of the CEC-32 cells are viable. In contrast 48h after infection the viability of the tumorigenic Hela cells is reduced under 10% independend of the used recombinant NDV.

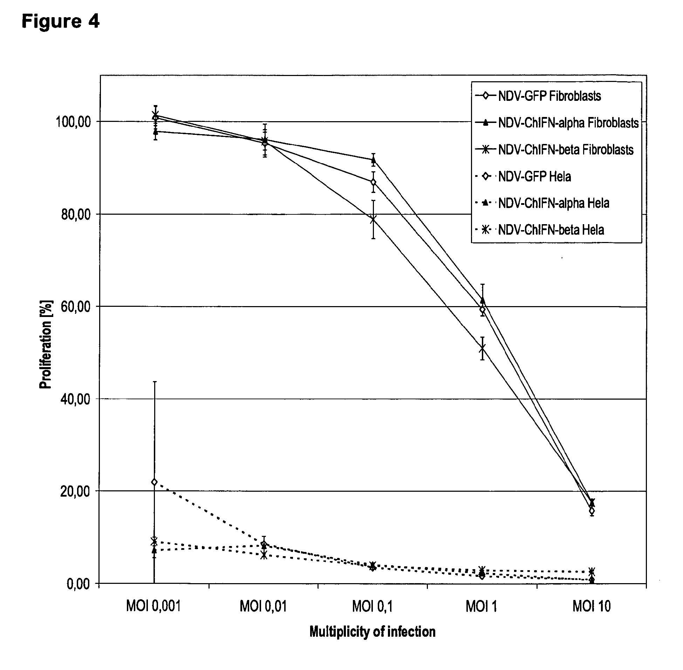

**Figure 4** shows that a large therapeutic window exists after the infection of tumor and fibroblast cells with NDV-ChIFN-alpha and NDV-ChIFN-beta. The proliferation inhibition of the oncolytic viruses NDV-ChIFN-alpha and NDV-ChIFN-beta is very strong on tumor cells and in contrast nearly no growth inhibition is observed after infection of primary fibroblast cells, especially at low MOIs like 0.1 and MOI 0.01.

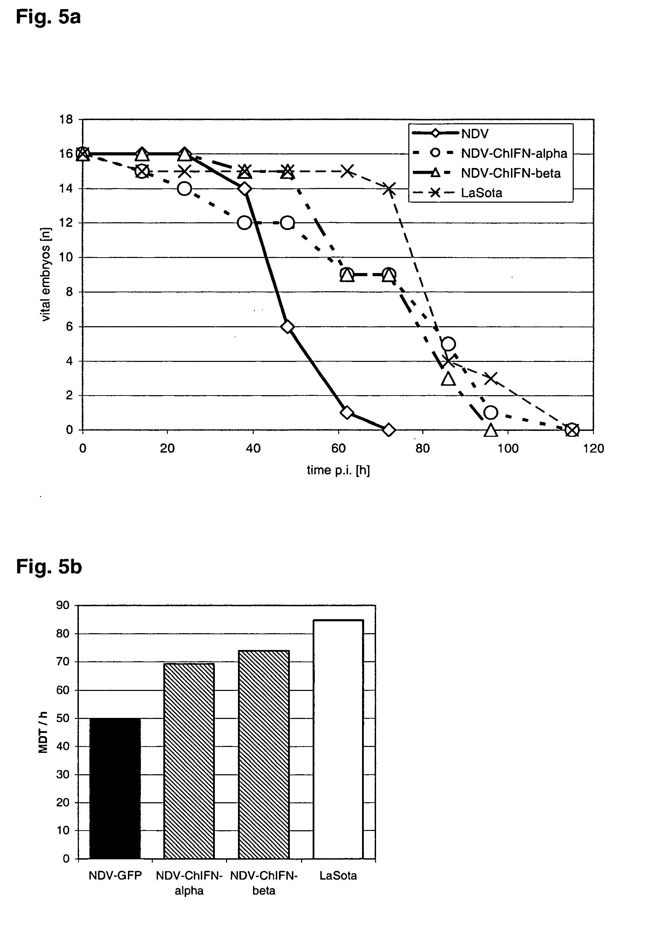

**Figure 5a** depicts the survival curves of NDV infected embryonated chicken eggs with the four viruses NDV-GFP, NDV-ChIFN-alpha, NDV-ChIFN-beta and the apathogenic strain LaSota. Chick embryos infected with NDV-ChIFN-alpha or NDV-ChIFN-beta are surviving longer than the NDV-GFP infected embryos. The curves are shifted clearly in the direction of the survival curve of the lentogenic NDV LaSota strain.

**Figure 5b**: From the results of the survival curves a MDT (Mean Death Time) is calculated for each infection group. The MDT is increased for the viruses NDV-ChIFN-alpha and ChIFN-beta compared with NDV-GFP. The highest MDT was observed with the apathogenic strain LaSota.

**Example 1:**

**Generation of a recombinant NDV with an avian derived transgene that lead to the expression of a chicken cytokine**

The oncolytic strain MTH68 of NDV was used to obtain viral RNA. Using RT-PCR several fragments of cDNA were obtained and in a multi-step cloning procedure they were assembled into a full-genome cDNA that was cloned into the vector pX8δT (Schnell et al., 1994) yielding the plasmid pflMTH68. This vector can be used for transfection in order to rescue recombinant virus from a T7-polymerase expressing cell line.

One additional transcriptional cassette were cloned into the full-length genomic plasmid of NDV MTH68 (pflMTH68) between the genes encoding the F-protein and the HN-protein into the unique SfiI restriction site. The two DNA-oligonucleotides

Sfi fw (5'-aggccttaattaaccgacaacttaagaaaaaatacgggtagaacggcctgag-3', SEQ. ID. NO: 1) and

Sfi back (5'-aggccgttctacccgtatttttcttaagttgtcggttaattaaggcctctc-3', SEQ. ID. NO: 2)

were annealed and subsequently ligated into the SfiI-site of pfIMTH68.

Two DNA transgenes coding for ChIFN-alpha (NM_205427) and ChIFN-beta (NM_001024836) were amplified by PCR. As templates for the polymerase chain reaction ChIFN-alpha and ChIFN-beta expression constructs described in Schultz et al, 1995 and Sick et al, 1996 were used. For the amplification of the ChIFN-alpha transgene the following primer pair: ChIFN-alpha fw (5'-ccttaattaagccaccatggctgtgcctgcaagccc-3' SEQ. ID NO:3) and ChIFN-alpha-rev (5'-ccttaattaactaagtgcgcgtgttgcctgtg-3' SEQ. ID NO:4) were used and for the amplification of ChIFN-beta coding sequence the two primers Pacl ChIFN-beta fw (5'-ccttaattaacgcaccatgactgcaaaccatcagtctccagg-3' SEQ. ID NO:5) and ChIFN-beta-rev (5'-ccttaattaatcactgggtgttgagacgtttggatg-3' SEQ. ID NO:6) were used.

Each of the two chIFN-transgenes were cloned into the Pacl site of the plasmid pfIMTH68Pac, respectively. The total length of the genome was adjusted to be a multiple of 6 to follow the "rule of six" for the length of the viral genome. The sequence identity of the ChIFN-beta insert was confirmed by nucleotide sequencing. In the ChIFN-alpha insert one G to A nucleotide exchange in position 89 in comparision with the sequence NM_205427 was detected. Recombinant virus was rescued from T7-expressing cells transfected with the full-length viral genomic plasmid containing the genes for ChIFN-alpha (Fig. 1) or ChIFN-beta (Fig. 2) by a standard virus rescue technique. The resulting ChIFN-alpha-expressing virus was designated NDV-ChIFN-alpha and the ChIFN-beta expressing NDV was named NDV-ChIFN-beta. The viruses were cultivated either in tissue culture or in the allantoic fluid of chicken eggs to produce high titres.

**Example 2:**

**ChIFN-alpha and ChIFN-beta expressed from a recombinant NDV is biological active.**

**Materials and Methods:**

CEC-32 cells (avian quail cell line) or Hela cells (cervical cancer cell line) were seeded in a 6 well plate at $1x10^5$ cells/well. After becoming adherent the cells were infected using a MOI of 0.01 with the GFP-expressing control virus NDV-GFP, NDV-ChIFN-alpha, NDV-ChIFN-beta or MOCK. After 64 h the supernatants were harvested and infectious virus particles were inactivated by UV irradiation.

To demonstrate the biological activity of ChIFN-alpha or ChIFN-beta in the supernatant of the virus-infected cells, a chicken interferon-specific bioassay was used (Schwarz et al, JICR, 2005). The assay is based on the stable transfected quail cell line CEC-511 carrying a luciferase-gene controlled by the IFN-responsive chicken Mx promotor. The luciferase activity is induced when the CEC-511 indicator cells where incubated with ChIFN-alpha or ChIFN-beta. To perform this assay 15.000 CEC-511 cells were seeded in 96-well plate. 24 h after seeding the cells were treated with the UV-treated supernatant of the virus-infected Hela or CEC-32 cells. Cells were incubated with 75 μl of a 1:100 dilution of the respective supernatants. As positive controls, cells were incubated with a 1:1000 dilution of supernatant from 293T-cells transfected with an expression plasmid for ChIFN-alpha or ChIFN-beta (Sick et al, 1996) or only medium. After 6h incubation time the Steady-Glo® Luciferase Assay (Promega) was performed, following the protocol provided by the manufacturer. To determine mean values each datapoint was measured as triplicate.

**Results:**

64 h after infection the supernatants of the NDV-ChIFN-alpha and NDV-ChIFN-beta infected CEC-32 and Hela cells have a strong luciferase inducing activity on CEC-511 cells indicating virus-mediated expression of ChIFN-alpha and ChIFN-beta in the infected cells. In contrast supernatant of MOCK-infected cells or infected with a control virus expressing GFP show no luciferase-inducing activity on the CEC-511 indicator cell line. A 1:1000 dilution of supernatant containing recombinant ChIFN-alpha or ChIFN-beta also demonstrate a ChIFN-dependent Mx promotor inducing activity.

Tab. 1: Interferon activity in the supernatant of CEC-32 and Hela cells infected with recombinant NDVs expressing ChIFN-alpha or ChIFN-beta. IFN response is measured in indicator cells carrying an Mx promotor-controlled luciferase reporter gene. Measured values are given in relative luciferase counts.

| | CEC-32 | | | |
| | NDV-GFP | NDV-ChIFN-alpha | NDV-ChIFN-beta | MOCK |
|---|---|---|---|---|
| Mean value | 308 | 11991 | 8033 | 246 |
| Standard dev. | 86 | 283 | 423 | 27 |
| | | | | |
| | | | | |
| | Hela | | | |
| | NDV-GFP | NDV-ChIFN-alpha | NDV-ChIFN-beta | MOCK |
| Mean value | 237 | 11739 | 5095 | 290 |
| Standard dev. | 19 | 1835 | 78 | 39 |
| | | | | |
| | | | | |
| | ChIFN-alpha | ChIFN-beta | Medium | |
| Mean value | 4849 | 2128 | 237 | |
| Standard dev. | 107 | 15 | 21 | |

**Example 3:**

**Avian cells but not tumor cells infected with recombinant NDVs expressing ChIFN-alpha or ChIFN-beta are protected from viral lysis**

**Materials and Methods:**

**3 a.)** CEC-32 cells (avian quail cell line) or Hela cells (cervical cancer cell line) were seeded in a 6 well plate at 1x10$^5$ cells/well. After becoming adherent the cells were infected with a MOI of 0.01 either with the control virus NDV-GFP, NDV-ChIFN-alpha, NDV-ChIFN-beta or MOCK. After 48h the cells were fixed with 4% formaldehyde solution and stained with Giemsa solution. Subsequent photodocumention was performed with a Zeiss Axiophot imaging system.

**3 b.)** CEC-32 cells (avian quail cell line) or Hela cells (cervical cancer cell line) were seeded in a 96 well plate at 15.000 cells/well. After becoming adherent the cells were infected with a MOI of 0.01 either with the control virus NDV-GFP, NDV-ChIFN-alpha, NDV-ChIFN-beta or MOCK (= non-infected). After 48h incubation time the CellTiter-Glo® Luciferase Assay (Promega) was performed to measure cell viability, following the protocol provided by the manufacturer. To determine the mean values, each datapoint was generated from six independend values.

**Results:**

**Fig 3. a.)** After 48h the avian CEC-32 cells are lysed by the GFP expressing NDV (NDV-GFP). In contrast cells infected with the ChIFN-alpha expressing virus are partially protected from lysis. The NDV-ChIFN-alpha infected CEC-32 monolayer is to 25-50% intact. An even stronger protection is seen with NDV expressing ChIFN-beta. After 48h nearly 90-100% of the CEC-32 monolyer is intact and protected from viral lysis. The density of this monolayer is comparable with the monolayer of the MOCK infected CEC-32 cells.

Infection with the three viruses on the tumorigenic Hela cell line shows a comparable oncolytic activity between the viruses NDV-GFP, NDV-ChIFN-alpha and NDV-ChIFN-beta. After 48h the monolayer of Hela cells infected with NDV-GFP, NDV-ChIFN-alpha or NDV-ChIFN-beta is nearly completely destroyed. The cell monolayer of MOCK-infected Hela cells is intact, indicated by the blue color of Giemsa-stained cells.

**Fig 3. b)** In a second experiment cell viabilty was quantified 48h after infection with the ChIFN-expressing NDVs. The NDV-GFP infected avian CEC-32 cells showed only 3% remaining cell viability. In the NDV-ChIFN-alpha infected CEC-32 cells 26% of the remaining cells are intact. 48h after NDV-ChIFN-beta infection of the CEC-32 quail cell line 94% of the cells are viable and protected against viral lysis.

In contrast nearly any difference in cell destruction can be observed after infection with the three viruses NDV-GFP, NDV-ChIFN-alpha or NDV-ChIFN-beta on the tumorigenic Hela cell line. After 48 h the value for the viable cells is for all three viruses between 3%-9% indicating nearly a complete tumor cell destruction.

### Example 4:

**Selective oncolysis of tumor cells but not primary cells by recombinant NDV expressing ChIFN-alpha or ChIFN-beta.**

### Materials and Methods:

5000 primary fibroblast cells or 3000 Hela cells were seeded in each well of a 96 well plate. 16h after seeding virus dilutions of NDV-GFP (NDV-GFP), NDV-ChIFN-alpha (NDV-ChIFN-alpha) and NDV-ChIFN-beta (NDV-ChIFN-beta) were prepared. Fibroblast cells were infected with the indicated virus concentrations. As a negative control fibroblasts cells were MOCK infected. After 1 h the inocculum was removed, cells were washed with PBS and incubated with 200 $\mu$l cell culture medium for 4 days in the cell culture incubator. Afterwards the remaining cells were fixed with 4% formaldehyde solution and stained with crystal violet. The stain was solubilized with 10% glacial acetic acid and the absorbence of each well was measured at 595 nm in an ELISA Reader.

### Results:

Four days after infection the primary fibroblasts infected with the three viruses NDV-GFP, NDV-ChIFN-alpha and NDV-ChIFN-beta using MOIs between MOI 0,001 up to MOI 0,1 show nearly no proliferation inhibition (Fig. 4). The first signs of proliferation inhibition are only observed at MOIs where 1 or 10 viral particles per cell were used for infection. In contrast tumorigenic Hela cells infected with MOIs between MOI 0,001 and MOI 10 show a very strong proliferation inhibition. This results indicates that the recombinant NDVs have a very good therapeutic window especially at low MOIs like MOI 0,1 and MOI 0,001 where nearly no lytic effect is measured in the primary cells and a very strong growth inhibitory effect is measured in the tumorigenic Hela cells. No difference in the kill curve shape on tumor and primary cells is observed between the control virus NDV-GFP and the ChIFN-expressing viruses NDV-ChIFN-alpha and NDV-ChIFN-beta.

### Example 5:

**Prolonged survival time of chicken embryos infected with recombinant NDV expresssing ChIFN-alpha or ChIFN-beta**

### Materials and Methods:

Groups of fifteen 11d old embryonated chicken eggs were infected with NDV-ChIFN-alpha, NDV-ChIFN-beta, NDV-GFP or the non-pathogenic NDV strain LaSota, respectively. As infectious material egg-grown virus in allantoic fluid diluted with sterile PBS was used. An infectious dose of 4000 PFU in a total volume of 200 $\mu$l was applied. The inoculum was injected into the allantoic cavity. After infection the eggs were incubated at 37°C and 50%-60%.humidity in a egg breader. Eggs were candled two times a day at the indicated time points (Fig. 5) and embryos were checked for signs of vitality.

This assay allows to compare the pathogenicity of diffferent NDV strains by comparing the survival time of the infected embryos. The method is based on the "Mean Death Time of the Minimum Lethal Dose (MDT/MLD)" determination described in R.P. Hanson (1980). The protocol was modified in that way that not serial dilutions of the virus stock were inocculated in the eggs, instead of one defined virus dose injection with a concentration higher than the minimal lethal dose. For ethical reasons this assay modification reduces the number of embryos used for the test. The MDT is determined by the following formula:

$$MDT = ((no.\ dead\ at\ X\ hr)\ x\ (X\ hr) + (no.\ dead\ at\ Y\ hr)\ x\ (y\ hr)\ etc.)/total$$
$$number\ dead$$

**Results:**

The survival of the NDV-ChIFN-alpha and NDV-ChIFN-beta infected chick embryos is improved in comparision with the NDV-GFP infected embryos. The NDV-GFP infected embryonated eggs are losing signs of vitality between 24h and 62h, leading to a MDT (Mean Death Time) of 50h. The NDV-ChIFN-beta infected embryos are surviving up to 96h with a calculated MDT of 69h. A comparable survival time with a MDT of 74h is measured in the NDV-ChIFN-beta infected embryos. Most of the NDV-ChIFN-beta infected embryos are dying between 48h and 96h. The sensitivity of the assay is indicated by usage of the apathogenic or lentogenic strain LaSota. Even embryonated eggs inocculated with the NDV LaSota strain are dying between 62h to 115h with a calculated MDT of 85h. This experiments shows that the MDT of the mesogenic NDV MTH68 is shifted towards a lentogenic NDV strain.

**References**

Adams, G.P. and Weiner, L.M. (2005) Monoclonal antibody therapy of cancer. Nat Biotechnol, 23, 1147-1157.

Alexander, D.J. (1988) Newcastle disease. Kluwer Academic Publishers, Norwell, Massachusetts.

Allen, T.M. (2002) Ligand-targeted therapeutics in anticancer therapy. Nat Rev Cancer, 2, 750-763.

Amstutz, P., Binz, H.K., Parizek, P., Stumpp, M.T., Kohl, A., Grutter, M.G., Forrer, P. and Pluckthun, A. (2005) Intracellular kinase inhibitors selected from combinatorial libraries of designed ankyrin repeat proteins. J Biol Chem, 280, 24715-24722.

Antoniw, P., Springer, C.J., Bagshawe, K.D., Searle, F., Melton, R.G., Rogers, G.T., Burke, P.J. and Sherwood, R.F. (1990) Disposition of the prodrug 4-(bis (2-chloroethyl) amino) benzoyl-L-glutamic acid and its active parent drug in mice. Br J Cancer, 62, 909-914.

Bagshawe, K.D., Springer, C.J., Searle, F., Antoniw, P., Sharma, S.K., Melton, R.G. and Sherwood, R.F. (1988) A cytotoxic agent can be generated selectively at cancer sites. Br J Cancer, 58, 700-703.

Batra, J.K., Fitzgerald, D.J., Chaudhary, V.K. and Pastan, I. (1991) Single-chain immunotoxins directed at the human transferrin receptor containing Pseudomonas exotoxin A or diphtheria toxin: anti-TFR(Fv)-PE40 and DT388-anti-TFR(Fv). Mol Cell Biol, 11, 2200-2205.

Batra, J.K., Kasprzyk, P.G., Bird, R.E., Pastan, I. and King, C.R. (1992) Recombinant anti-erbB2 immunotoxins containing Pseudomonas exotoxin. Proc Natl Acad Sci U S A, 89, 5867-5871.

Bell, J.C., Garson, K.A., Lichty, B.D. and Stojdl, D.F. (2002) Oncolytic viruses: programmable tumor hunters. Curr Gene Ther, 2, 243-254.

Binz, H.K., Amstutz, P., Kohl, A., Stumpp, M.T., Briand, C., Forrer, P., Grutter, M.G. and Pluckthun, A. (2004) High-affinity binders selected from designed ankyrin repeat protein libraries. Nat Biotechnol, 22, 575-582.

Bosslet, K., Czech, J., Lorenz, P., Sedlacek, H.H., Schuermann, M. and Seemann, G. (1992) Molecular and functional characterisation of a fusion protein suited for tumor specific prodrug activation. Br J Cancer, 65, 234-238.

Carnemolla, B., Borsi, L., Balza, E., Castellani, P., Meazza, R., Berndt, A., Ferrini, S., Kosmehl, H., Neri, D. and Zardi, L. (2002) Enhancement of the antitumor properties of interleukin-2 by its targeted delivery to the tumor blood vessel extracellular matrix. Blood, 99, 1659-1665.

Chaudhary, V.K., Gallo, M.G., FitzGerald, D.J. and Pastan, I. (1990) A recombinant single-chain immunotoxin composed of anti-Tac variable regions and a truncated diphtheria toxin. Proc Natl Acad Sci U S A, 87, 9491-9494.

Chaudhary, V.K., Queen, C., Junghans, R.P., Waldmann, T.A., FitzGerald, D.J. and Pastan, I. (1989) A recombinant immunotoxin consisting of two antibody variable domains fused to Pseudomonas exotoxin. Nature, 339, 394-397.

Chiocca, E.A. (2002) Oncolytic viruses. Nat Rev Cancer, 2, 938-950.

Csatary, L.K., Gosztonyi, G., Szeberenyi, J., Fabian, Z., Liszka, V., Bodey, B. and Csatary, C.M. (2004) MTH-68/H oncolytic viral treatment in human high-grade gliomas. J Neurooncol, 67, 83-93.

De Lorenzo, C., Arciello, A., Cozzolino, R., Palmer, D.B., Laccetti, P., Piccoli, R. and D'Alessio, G. (2004) A fully human antitumor immunoRNase selective for ErbB-2-positive carcinomas. Cancer Res, 64, 4870-4874.

Engel-Herbert, I., Werner, O., Teifke, J.P., Mebatsion, T., Mettenleiter, T.C. and Romer-Oberdorfer, A. (2003) Characterization of a recombinant Newcastle disease virus expressing the green fluorescent protein. J Virol Methods, 108, 19-28.

Ferrara, N., Hillan, K.J., Gerber, H.P. and Novotny, W. (2004) Discovery and development of bevacizumab, an anti-VEGF antibody for treating cancer. Nat Rev Drug Discov, 3, 391-400.

Ford, K.G., Souberbielle, B.E., Darling, D. and Farzaneh, F. (2001) Protein transduction: an alternative to genetic intervention? Gene Ther, 8, 1-4.

Georgakis, G.V., Li, Y., Humphreys, R., Andreeff, M., O'Brien, S., Younes, M., Carbone, A., Albert, V. and Younes, A. (2005) Activity of selective fully human agonistic antibodies to the TRAIL death receptors TRAIL-R1 and TRAIL-R2 in primary and cultured lymphoma cells: induction of apoptosis and enhancement of doxorubicin- and bortezomib-induced cell death. Br J Haematol, 130, 501-510.

Gerber, H.P. and Ferrara, N. (2005) Pharmacology and pharmacodynamics of bevacizumab as monotherapy or in combination with cytotoxic therapy in preclinical studies. Cancer Res, 65, 671-680.

Hanahan, D. and Weinberg, R.A. (2000) The hallmarks of cancer. Cell, 100, 57-70.

Hanson, R.P: (1980) Newcastle Disease. In Hitchner, S. B. (ed.). Isolation and identification of avian pathogens. College Station, Tex: American Association of Avian Pathologists.

Herbst, R.S. and Langer, C.J. (2002) Epidermal growth factor receptors as a target for cancer treatment: the emerging role of IMC-C225 in the treatment of lung and head and neck cancers. Semin Oncol, 29, 27-36.

Hogbom, M., Eklund, M., Nygren, P.A. and Nordlund, P. (2003) Structural basis for recognition by an in vitro evolved affibody. Proc Natl Acad Sci U S A, 100, 3191-3196.

Huang, Z., Elankumaran, S., Panda, A. and Samal, S.K. (2003) Recombinant Newcastle disease virus as a vaccine vector. Poult Sci. 82, 899-906.

Huang, Z., Elankumaran, S., Yunus, A.S. and Samal, S.K. (2004) A recombinant Newcastle disease virus (NDV) expressing VP2 protein of infectious bursal disease virus (IBDV) protects against NDV and IBDV. J Virol, 78, 10054-10063.

Huang, Z., Krishnamurthy, S., Panda, A. and Samal, S.K. (2001) High-level expression of a foreign gene from the most 3'-proximal locus of a recombinant Newcastle disease virus. J Gen Virol, 82, 1729-1736.

Kreitman, R.J., Chaudhary, V.K., Waldmann, T.A., Hanchard, B., Cranston, B., FitzGerald, D.J. and Pastan, I. (1993) Cytotoxic activities of recombinant immunotoxins composed of Pseudomonas toxin or diphtheria toxin toward lymphocytes from patients with adult T-cell leukemia. Leukemia, 7, 553-562.

Kaiser, P" Poh, T.Y., Rothwell, L., Avery, S., Balu, S., Pathania, U.S., Hughes, S., Goodchild, M., Morrell, S., Watson, M., Bumstead, N., Kaufman, J. and Young, J.R. (2005) A genomic analysis of chicken cytokines and chemokines. J Interferon Cytokine Res. 25: 467-484.

Kreitman, R.J. and Pastan, I. (1995) Importance of the glutamate residue of KDEL in increasing the cytotoxicity of Pseudomonas exotoxin derivatives and for increased binding to the KDEL receptor. Biochem J, 307 (Pt1), 29-37.

Krishnamurthy, S., Huang, Z. and Samal, S.K. (2000) Recovery of a virulent strain of newcastle disease virus from cloned cDNA: expression of a foreign gene results in growth retardation and attenuation. Virology, 278, 168-182.

Kuan, S.F., Byrd, J.C., Basbaum, C.B. and Kim, Y.S. (1987) Characterization of quantitative mucin variants from a human colon cancer cell line. Cancer Res, 47, 5715-5724.

Ladner, R.C. and Ley, A.C. (2001) Novel frameworks as a source of high-affinity ligands. Curr Opin Biotechnol, 12, 406-410.

Lamb, R.A., Kolakofsky, D. (2001) Paramyxoviridae: The Viruses And Their Replication. In Fields, B.N. (ed.), Virology. Lippincott Williams & Wilkins, pp. 1305-1485.

Lorence, R.M., Pecora, A.L., Major, P.P., Hotte, S.J., Laurie, S.A., Roberts, M.S., Groene, W.S. and Bamat, M.K. (2003) Overview of phase I studies of intravenous administration of PV701, an oncolytic virus. Curr Opin Mol Ther, 5, 618-624.

Mizukami, Y., Jo, W.S., Duerr, E.M., Gala, M., Li, J., Zhang, X., Zimmer, M.A., Iliopoulos, O., Zukerberg, L.R., Kohgo, Y., Lynch, M.P., Rueda, B.R. and Chung, D.C. (2005) Induction of interleukin-8 preserves the angiogenic response in HIF-1alpha-deficient colon cancer cells. Nat Med, 11, 992-997.

Nakaya, T., Cros, J., Park, M.S., Nakaya, Y., Zheng, H., Sagrera, A., Villar, E., Garcia-Sastre, A. and Palese, P. (2001) Recombinant Newcastle disease virus as a vaccine vector. J Virol, 75, 11868-11873.

Noonberg, S.B. and Benz, C.C. (2000) Tyrosine kinase inhibitors targeted to the epidermal growth factor receptor subfamily: role as anticancer agents. Drugs, 59, 753-767.

Nord, K., Gunneriusson, E., Uhl inverted question marken, M. and Nygren, P.A. (2000) Ligands selected from combinatorial libraries of protein A for use in affinity capture of apolipoprotein A-1M and taq DNA polymerase. J Biotechnol, 80, 45-54.

Roffler, S.R., Wang, S.M., Chern, J.W., Yeh, M.Y. and Tung, E. (1991) Antineoplastic glucuronide prodrug treatment of human tumor cells targeted with a monoclonal antibody-enzyme conjugate. Biochem Pharmacol, 42, 2062-2065.

Romer-Oberdorfer, A., Mundt, E., Mebatsion, T., Buchholz, U.J. and Mettenleiter, T.C. (1999) Generation of recombinant lentogenic Newcastle disease virus from cDNA. J Gen Virol, 80 ( Pt 11), 2987-2995.

Rowlinson-Busza, G., Bamias, A., Krausz, T. and Epenetos, A.A. (1992) Antibody guided enzyme nitrile therapy (Agent): in vitro cytotoxicity and in vivo tumor localisation. In Epenetos, a. (ed.), Monoclonal Antibodies 2. Applications in Clinical Oncology. Chapman and Hall, London, pp. 111-118.

Russell, S.J. (2002) RNA viruses as virotherapy agents. Cancer Gene Ther, 9, 961-966.

Salgaller, M.L. (2003) Technology evaluation: bevacizumab, Genentech/Roche. Curr Opin Mol Ther, 5, 657-667.

Schnell, M.J., Mebatsion, T. and Conzelmann, K.K. (1994) Infectious rabies viruses from cloned cDNA. Embo J, 13, 4195-4203.

Schultz, U., Rinderle, C., Sekellick, M.J., Marcus, P.I. and Staeheli, P. (1995) Recombinant chicken interferon from Escherichia coli and transfected COS cells is biologically active..Eur J Biochem. 229:73-76.

Schwarz, H., Harlin, O., Ohnemus, A., Kaspers, B. and Staeheii, P. (2004) Synthesis of IFN-beta by virus-infected chicken embryo cells demonstrated with specific antisera and a new bioassay. J Interferon Cytokine Res. 24:179-184.

Sick, C., Schultz, U. and Staeheli, P. (1996) A family of genes coding for two serologically distinct chicken interferons. J Biol Chem., 271, 7635-7639.

Sinkovics, J.G. and Horvath, J.C. (2000) Newcastle disease virus (NDV): brief history of its oncolytic strains. J Clin Virol, 16, 1-15.

Skerra, A. (2000) Lipocalins as a scaffold. Biochim Biophys Acta, 1482, 337-350.

Staeheli, P., Puehler, F., Schneider, K., Gobel, T.W. and Kaspers, B. (2001) Cytokines of birds: conserved functions-- a largely different look. J Interferon Cytokine Res. 2001 21:993-1010.

Stojdl, D.F., Lichty, B.D., tenOever, B.R., Paterson, J.M., Power, A.T., Knowles, S., Marius, R., Reynard, J., Poliquin, L., Atkins, H., Brown, E.G., Durbin, R.K., Durbin, J.E., Hiscott, J. and Bell, J.C. (2003) VSV strains with defects in their ability to shutdown innate immunity are potent systemic anti-cancer agents. Cancer Cell, 4, 263-275.

Vogelstein, B. and Kinzler, K.W. (2004) Cancer genes and the pathways they control. Nat Med, 10, 789-799.

Waltenberger, J., Claesson-Welsh, L., Siegbahn, A., Shibuya, M. and Heldin, C.H. (1994) Different signal transduction properties of KDR and Flt1, two receptors for vascular endothelial growth factor. J Biol Chem, 269, 26988-26995.

Wang, S.M., Chern, J.W., Yeh, M.Y., Ng, J.C., Tung, E. and Roffler, S.R. (1992) Specific activation of glucuronide prodrugs by antibody-targeted enzyme conjugates for cancer therapy. Cancer Res, 52, 4484-4491.

Warren, R.S., Yuan, H., Matli, M.R., Gillett, N.A. and Ferrara, N. (1995) Regulation by vascular endothelial growth factor of human colon cancer tumorigenesis in a mouse model of experimental liver metastasis. J Clin Invest, 95, 1789-1797.

Zewe, M., Rybak, S.M., Dubel, S., Coy, J.F., Welschof, M., Newton, D.L. and Little, M. (1997) Cloning and cytotoxicity of a human pancreatic RNase immunofusion. Immunotechnology, 3, 127-136.

Zhao, H. and Peeters, B.P. (2003) Recombinant Newcastle disease virus as a viral vector: effect of genomic location of foreign gene on gene expression and virus replication. J Gen Virol, 84, 781-788.

**Claims**

1. A recombinant oncolytic RNA Newcastle Disease Virus comprising at least one transgene coding for an avian cytokine, wherein the recombinant oncolytic RNA Newcastle Disease Virus is obtainable from a velogenic or mesogenic oncolytic RNA Newcastle Disease Virus.

2. The virus according to claim 1, comprising at least one further transgene having therapeutic activity when expressed by a virus-infected tumor cell.

3. The virus according to claim 2, wherein the at least one further transgene is partially allogene or syngene for the host.

4. The virus according to any of the claims 1 to 3, wherein the pathogenicity of the virus is reduced for an avian species.

5. The virus of claim 4, wherein the pathogenicity of the virus is reduced for an avian species with respect to the virus from which the recombinant virus is obtainable.

6. The virus of claim 4 or 5, wherein the avian species is selected from poultry.

7. The virus of any of the claims 4 to 6, wherein the avian species is chicken.

8. The virus of any of the claims 1 to 7, which is an avian pathogen, in particular a pathogen of poultry, more particular a pathogen of chicken.

9. The virus of any of the claims 1 to 8, obtainable from the mesogenic strain MTH68.

10. The virus of any of the claims 1 to 9, wherein the at least one further transgene codes for a binding protein that has a therapeutic activity when expressed by the virus-infected tumor cell.

11. The virus according to claim 10, wherein the binding protein is selected from the following group consisting of a natural ligand, a genetically modified ligand, a recombinant soluble domain of a natural receptor and a modified version thereof, a peptide ligand, a polypeptide ligand, an antibody molecule and fragments and derivatives thereof, and an antibody-like molecule like an ankyrin-repeat protein and fragments and derivatives thereof.

12. The virus according to claim 10 or 11, wherein the binding protein is of mammalian, e.g. human, murine or closely related origin or a chimeric protein.

13. The virus according to any of one the claims 10 to 12, wherein the binding protein is a monomeric, dimeric, trimeric, tetrameric or multimeric protein.

14. The virus according to any one of the claims 10 to 13, wherein the binding protein is monospecific, bispecific or multispecific.

15. The virus according to any one of the claims 10 to 14, wherein the binding protein is a fusion protein comprising at least one binding domain and at least one heterologous domain.

16. The virus according to claim 15, wherein the binding protein is a fusion protein comprising a toxin such as human RNAse (pseudomonas exotoxin, Diphtheria toxin), or a fusion protein comprising an enzyme like beta-glucuronidase, beta-galactosidase, beta-glucosidase, carboxypeptidase, beta-lactamase, or a fusion protein comprising an immune-stimulatory protein with cytokine activity like IL-2, IL-12, TNF-alpha, IFN-beta or GM-CSF.

17. The virus according to any one of the claims 10 to 16, wherein the binding protein is selected from the group constisting of blocking proteins of autonomous active growth factor receptors (eg. EGFR, Met), competitive binders for growth factors (antagonists), blocking proteins for Rb-phosphorylation, blocking proteins for E2F-dependent transcription; stabilizers for p53; antagonistic binders for antiapoptotic proteins (eg. Bcl-2); antagonistic binders for cyclins; antagonistic binders for Ras effectors (eg. GEFs); antagonistic binders for hypoxia induced proteins (eg. HIF1$\alpha$); inhibitors of transcription factors that interfere with dimerization, DNA-binding or/and cofactor binding (eg. Myc/Max); Inducers of differentiation; Inhibitors of smad signalling/translocation; inhibitors of cellular adhesion interactions (cadherins, integrins, eg. $\alpha5\beta1$, $\alpha v\beta3$); inhibitors of enzymes that degrade the extracellular matrix (eg. MMPs); antagonistic binders for proangiogenic ligands (eg. soluble VEGF-R); antagonistic binders to proangiogenic receptors; inhibitors of scaffold complex formation (eg. KSR/Ras); inhibitors of translation initiation (eg. eIF4E, EIF2a); and inhibitors of mitotic kinases (eg. PIk-1).

18. The virus of any of the claims 1 to 9, wherein the at least one further transgene codes for a prodrug-converting enzyme that has a therapeutic activity when expressed by the virus-infected tumor cell.

19. The virus of any of the claims 1 to 9, wherein the at least one further transgene codes for a protease that has a therapeutic activity when expressed by the virus-infected tumor cell.

20. The virus of any of the preceding claims, wherein the at least one transgene encoding an avian cytokine is selected from chicken interferons.

21. The virus of claim 20, wherein the at least one transgene encoding an avian cytokine is selected from chicken type I interferons.

22. The virus of any of the claims 5 to 21, wherein the pathogenicity reduction is a capability of the virus to reduce bird cell lysis about 48h after infection with MOI 0,01 measured by increasing cell viability, whereby the cell viability is inceased to at least about 25% up to about 50% surviving cells, more preferably to at least about 50% up to about 75% surviving cells and most preferably to at least about 75% up to 100% surviving cells with respect to the virus from which the recombinant virus is obtainable.

23. The virus of any of the claims 5 to 21, wherein the pathogenicity reduction is a survival time prolongation of virus infected chicken embryos in 11 day old embryonated eggs measured by mean death time (MDT) determination, whereby the MDT is prolonged by at least about 15h up to about 20h, more preferably at least about 20h up to about 30h and most preferably by more than 30h compared to the virus from which the recombinant virus is obtainable.

24. The virus of any of the claims 1 to 23, wherein the oncolytic activity of the virus for human tumor cells is essentially not reduced.

25. The virus of any of the claims 1 to 23, wherein the oncolytic activity of the virus for human tumor cells measured by cell viability after 48h after infection is not reduced by more than 50% compared to the virus from which the recombinant virus is obtainable and more preferably is essentially not reduced with respect to the virus from which the recombinant virus is obtainable.

26. A nucleocapsid of a recombinant oncolytic RNA virus of any one of claims 1-25.

27. A genome of a recombinant oncolytic RNA virus of any one of claims 1-25.

28. A DNA molecule encoding the genome and/or antigenome of a recombinant oncolytic RNA virus of any one of the claims 1-25.

29. The DNA molecule of claim 27 operatively linked to a transcriptional control sequence.

30. A cell comprising a recombinant oncolytic virus of any one of claims 1-25, a virus genome of claims 27 or/and a DNA molecule of claim 28 or 29.

31. A pharmaceutical composition comprising a recombinant oncolytic virus of any one of claims 1-25, a virus genome of claims 27 or/and a DNA molecule of claim 28 or 29 as an active ingredient optionally together with pharmaceutically acceptable carriers, diluents and/or adjuvants.

32. The pharmaceutical composition of claim 31 further comprising a prodrug which can be converted into a therapeutically active compound by the prodrug-converting enzyme encoded by the at least one further transgene.

33. A method for treatment of a proliferative disease, comprising administering in a pharmaceutically effective amount to a subject in need thereof a recombinant oncolytic virus of any one of claims 1-25, a virus genome of claim 27 or/and a DNA molecule of claim 28 or 29.

34. The method of claim 33, comprising administering in a pharmaceutically effective amount to a subject in need thereof

(i) a recombinant oncolytic virus of any one of claims 1-25, a virus genome of claims 27 or/and a DNA molecule of claim 28 or 29 comprising at least one transgene encoding for a prodrug-converting enzyme and
(ii) a prodrug suitable for treatment of the proliferative disease in combination with the virus, which prodrug can be converted into a pharmaceutically active compound by the prodrug-converting enzyme of (i).

35. The method of any of the claims 33 to 34, wherein the subject is a human patient.

36. A recombinant oncolytic RNA virus attenuated for poultry comprising a nucleic acid comprising at least one transgene coding for a cytokine.

37. The virus of claim 36 which is attenuated for chicken.

# Fig. 1:

**plasmid pflMTH68 ChIFN-alpha**

**Fig. 2:**

**plasmid pflMTH68 ChIFN-beta**

**Fig. 3a**

avian cells

| MOCK | NDV-GFP |
| --- | --- |

NDV-ChIFN-α          NDV-ChIFN-β

**48h after infection**

tumor cells

| MOCK | NDV-GFP |
| --- | --- |

NDV-ChIFN-α          NDV-ChIFN-β

**48h after infection**

## Figure 3b

## Figure 4

## Fig. 5a

## Fig. 5b

**European Patent Office**

## EUROPEAN SEARCH REPORT

Application Number

EP 08 00 1643

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | US 2003/224017 A1 (SAMAL SIBA K [US] ET AL) 4 December 2003 (2003-12-04) <br><br> * the whole document * <br> ----- | 1-8, 22-31, 36,37 | INV. A61K35/76 |
| X | WO 00/62735 A (PRO VIRUS INC [US]; ROBERTS MICHAEL S [US]; LORENCE ROBERT M [US]; GRO) 26 October 2000 (2000-10-26) <br> * sentence 7 - sentence 10 * <br> ----- | 26,36,37 | |
| X | WO 2006/050984 A (SCHERING AG [DE]; BEIER RUDOLF [DE]; PUEHLER FLORIAN [DE]) 18 May 2006 (2006-05-18) <br> * the whole document * <br> ----- | 26,36,37 | |
| X | LORENCE R M ET AL: "REPLICATION-COMPETENT, ONCOLYTIC NEWCASTLE DISEASE VIRUS FOR CANCER THERAPY" MONOGRAPHS IN VIROLOGY, KARGER, BASEL, CH, vol. 22, 1 January 2001 (2001-01-01), pages 160-182, XP008051134 ISSN: 0077-0965 * the whole document * <br> ----- | 26 | |
| A | MO C W ET AL: "THE IN VIVO AND IN VITRO EFFECTS OF CHICKEN INTERFERON ALPHA ON INFECTIOUS BURSAL DISEASE VIRUS AND NEWCASTLE DISEASE VIRUS INFECTION" AVIAN DISEASES, AMERICAN ASSOCIATION OF AVIAN PATHOLOGISTS, KENNET SQ., PA, US, vol. 45, no. 2, 1 April 2001 (2001-04-01), pages 389-399, XP008069611 ISSN: 0005-2086 * the whole document * <br> ----- | 1-37 | |

**TECHNICAL FIELDS SEARCHED (IPC)**

A61K
C12N

The present search report has been drawn up for all claims

| Place of search | Date of completion of the search | Examiner |
|---|---|---|
| Munich | 27 August 2008 | Wimmer, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 08 00 1643

This annex lists the patent family members relating to the patent documents cited in the above-mentioned European search report.
The members are as contained in the European Patent Office EDP file on
The European Patent Office is in no way liable for these particulars which are merely given for the purpose of information.

27-08-2008

| Patent document cited in search report | Publication date | Patent family member(s) | | Publication date |
|---|---|---|---|---|
| US 2003224017 A1 | 04-12-2003 | NONE | | |
| WO 0062735 A | 26-10-2000 | AU | 4246900 A | 02-11-2000 |
| | | CA | 2370187 A1 | 26-10-2000 |
| | | CN | 1477964 A | 25-02-2004 |
| | | EP | 1390046 A2 | 25-02-2004 |
| | | HU | 0302278 A2 | 28-10-2003 |
| | | JP | 2003530301 T | 14-10-2003 |
| | | MX | PA01010393 A | 02-04-2004 |
| WO 2006050984 A | 18-05-2006 | AU | 2005303912 A1 | 18-05-2006 |
| | | CA | 2585435 A1 | 18-05-2006 |
| | | CN | 101056646 A | 17-10-2007 |
| | | JP | 2008519590 T | 12-06-2008 |
| | | KR | 20070085314 A | 27-08-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030044384 A
- US 6699479 B1
- US 20040043035 A
- US 20030224017 A
- EP 1300157 A
- US 6719979 B
- WO 2007025431 A
- WO 2000067786 A
- EP 0702085 A
- WO 9966045 A
- WO 0062735 A
- WO 0120989 A
- US 0026116 W
- WO 03005964 A
- US 20040170607 A
- DE 19932688
- US 20050175581 A
- US 20040072276 A
- WO 2006050984 A

### Non-patent literature cited in the description

- Remington's Pharmaceutical Science. Mack Publishing Company, 1980
- Mean Death Time of the Minimum Lethal Dose. *R.P. Hanson,* 1980
- **Adams, G.P. ; Weiner, L.M.** Monoclonal antibody therapy of cancer. *Nat Biotechnol,* 2005, vol. 23, 1147-1157
- **Alexander, D.J.** Newcastle disease. Kluwer Academic Publishers, 1988
- **Allen, T.M.** Ligand-targeted therapeutics in anticancer therapy. *Nat Rev Cancer,* 2002, vol. 2, 750-763
- **Amstutz, P. ; Binz, H.K. ; Parizek, P. ; Stumpp, M.T. ; Kohl, A. ; Grutter, M.G. ; Forrer, P. ; Pluckthun, A.** Intracellular kinase inhibitors selected from combinatorial libraries of designed ankyrin repeat proteins. *J Biol Chem,* 2005, vol. 280, 24715-24722
- **Antoniw, P. ; Springer, C.J. ; Bagshawe, K.D. ; Searle, F. ; Melton, R.G. ; Rogers, G.T. ; Burke, P.J. ; Sherwood, R.F.** Disposition of the prodrug 4-(bis (2-chloroethyl) amino) benzoyl-L-glutamic acid and its active parent drug in mice. *Br J Cancer,* 1990, vol. 62, 909-914
- **Bagshawe, K.D. ; Springer, C.J. ; Searle, F. ; Antoniw, P. ; Sharma, S.K. ; Melton, R.G. ; Sherwood, R.F.** A cytotoxic agent can be generated selectively at cancer sites. *Br J Cancer,* 1988, vol. 58, 700-703
- **Batra, J.K. ; Fitzgerald, D.J. ; Chaudhary, V.K. ; Pastan, I.** Single-chain immunotoxins directed at the human transferrin receptor containing Pseudomonas exotoxin A or diphtheria toxin: anti-TFR(Fv)-PE40 and DT388-anti-TFR(Fv). *Mol Cell Biol,* 1991, vol. 11, 2200-2205
- **Batra, J.K. ; Kasprzyk, P.G. ; Bird, R.E. ; Pastan, I. ; King, C.R.** Recombinant anti-erbB2 immunotoxins containing Pseudomonas exotoxin. *Proc Natl Acad Sci U S A,* 1992, vol. 89, 5867-5871
- **Bell, J.C. ; Garson, K.A. ; Lichty, B.D. ; Stojdl, D.F.** Oncolytic viruses: programmable tumor hunters. *Curr Gene Ther,* 2002, vol. 2, 243-254
- **Binz, H.K. ; Amstutz, P. ; Kohl, A. ; Stumpp, M.T. ; Briand, C. ; Forrer, P. ; Grutter, M.G. ; Pluckthun, A.** High-affinity binders selected from designed ankyrin repeat protein libraries. *Nat Biotechnol,* 2004, vol. 22, 575-582
- **Bosslet, K. ; Czech, J. ; Lorenz, P. ; Sedlacek, H.H. ; Schuermann, M. ; Seemann, G.** Molecular and functional characterisation of a fusion protein suited for tumor specific prodrug activation. *Br J Cancer,* 1992, vol. 65, 234-238
- **Carnemolla, B. ; Borsi, L. ; Balza, E. ; Castellani, P. ; Meazza, R. ; Berndt, A. ; Ferrini, S. ; Kosmehl, H. ; Neri, D. ; Zardi, L.** Enhancement of the antitumor properties of interleukin-2 by its targeted delivery to the tumor blood vessel extracellular matrix. *Blood,* 2002, vol. 99, 1659-1665
- **Chaudhary, V.K. ; Gallo, M.G. ; FitzGerald, D.J. ; Pastan, I.** A recombinant single-chain immunotoxin composed of anti-Tac variable regions and a truncated diphtheria toxin. *Proc Natl Acad Sci U S A,* 1990, vol. 87, 9491-9494
- **Chaudhary, V.K. ; Queen, C. ; Junghans, R.P. ; Waldmann, T.A. ; FitzGerald, D.J. ; Pastan, I.** A recombinant immunotoxin consisting of two antibody variable domains fused to Pseudomonas exotoxin. *Nature,* 1989, vol. 339, 394-397
- **Chiocca, E.A.** Oncolytic viruses. *Nat Rev Cancer,* 2002, vol. 2, 938-950

- **Csatary, L.K. ; Gosztonyi, G. ; Szeberenyi, J. ; Fabian, Z. ; Liszka, V. ; Bodey, B. ; Csatary, C.M.** MTH-68/H oncolytic viral treatment in human high-grade gliomas. *J Neurooncol,* 2004, vol. 67, 83-93

- **De Lorenzo, C. ; Arciello, A. ; Cozzolino, R. ; Palmer, D.B. ; Laccetti, P. ; Piccoli, R. ; D'Alessio, G.** A fully human antitumor immunoRNase selective for ErbB-2-positive carcinomas. *Cancer Res,* 2004, vol. 64, 4870-4874

- **Engel-Herbert, I. ; Werner, O. ; Teifke, J.P. ; Mebatsion, T. ; Mettenleiter, T.C. ; Romer-Oberdorfer, A.** Characterization of a recombinant Newcastle disease virus expressing the green fluorescent protein. *J Virol Methods,* 2003, vol. 108, 19-28

- **Ferrara, N. ; Hillan, K.J. ; Gerber, H.P. ; Novotny, W.** Discovery and development of bevacizumab, an anti-VEGF antibody for treating cancer. *Nat Rev Drug Discov,* 2004, vol. 3, 391-400

- **Ford, K.G. ; Souberbielle, B.E. ; Darling, D. ; Farzaneh, F.** Protein transduction: an alternative to genetic intervention?. *Gene Ther,* 2001, vol. 8, 1-4

- **Georgakis, G.V. ; Li, Y. ; Humphreys, R. ; Andreeff, M. ; O'Brien, S. ; Younes, M. ; Carbone, A. ; Albert, V. ; Younes, A.** Activity of selective fully human agonistic antibodies to the TRAIL death receptors TRAIL-R1 and TRAIL-R2 in primary and cultured lymphoma cells: induction of apoptosis and enhancement of doxorubicin- and bortezomib-induced cell death. *Br J Haematol,* 2005, vol. 130, 501-510

- **Gerber, H.P. ; Ferrara, N.** Pharmacology and pharmacodynamics of bevacizumab as monotherapy or in combination with cytotoxic therapy in preclinical studies. *Cancer Res,* 2005, vol. 65, 671-680

- **Hanahan, D. ; Weinberg, R.A.** The hallmarks of cancer. *Cell,* 2000, vol. 100, 57-70

- Newcastle Disease. **Hanson, R.P.** Isolation and identification of avian pathogens. American Association of Avian Pathologists, 1980

- **Herbst, R.S. ; Langer, C.J.** Epidermal growth factor receptors as a target for cancer treatment: the emerging role of IMC-C225 in the treatment of lung and head and neck cancers. *Semin Oncol,* 2002, vol. 29, 27-36

- **Hogbom, M. ; Eklund, M. ; Nygren, P.A. ; Nordlund, P.** Structural basis for recognition by an in vitro evolved affibody. *Proc Natl Acad Sci U S A,* 2003, vol. 100, 3191-3196

- **Huang, Z. ; Elankumaran, S. ; Panda, A. ; Samal, S.K.** Recombinant Newcastle disease virus as a vaccine vector. *Poult Sci.,* 2003, vol. 82, 899-906

- **Huang, Z. ; Elankumaran, S. ; Yunus, A.S. ; Samal, S.K.** A recombinant Newcastle disease virus (NDV) expressing VP2 protein of infectious bursal disease virus (IBDV) protects against NDV and IBDV. *J Virol,* 2004, vol. 78, 10054-10063

- **Huang, Z. ; Krishnamurthy, S. ; Panda, A. ; Samal, S.K.** High-level expression of a foreign gene from the most 3'-proximal locus of a recombinant Newcastle disease virus. *J Gen Virol,* 2001, vol. 82, 1729-1736

- **Kreitman, R.J. ; Chaudhary, V.K. ; Waldmann, T.A. ; Hanchard, B. ; Cranston, B. ; FitzGerald, D.J. ; Pastan, I.** Cytotoxic activities of recombinant immunotoxins composed of Pseudomonas toxin or diphtheria toxin toward lymphocytes from patients with adult T-cell leukemia. *Leukemia,* 1993, vol. 7, 553-562

- **Kaiser, P ; Poh, T.Y. ; Rothwell, L. ; Avery, S. ; Balu, S. ; Pathania, U.S. ; Hughes, S. ; Goodchild, M. ; Morrell, S. ; Watson, M.** A genomic analysis of chicken cytokines and chemokines. *J Interferon Cytokine Res.,* 2005, vol. 25, 467-484

- **Kreitman, R.J. ; Pastan, I.** Importance of the glutamate residue of KDEL in increasing the cytotoxicity of Pseudomonas exotoxin derivatives and for increased binding to the KDEL receptor. *Biochem J,* 1995, vol. 307 (1), 29-37

- **Krishnamurthy, S. ; Huang, Z. ; Samal, S.K.** Recovery of a virulent strain of newcastle disease virus from cloned cDNA: expression of a foreign gene results in growth retardation and attenuation. *Virology,* 2000, vol. 278, 168-182

- **Kuan, S.F. ; Byrd, J.C. ; Basbaum, C.B. ; Kim, Y.S.** Characterization of quantitative mucin variants from a human colon cancer cell line. *Cancer Res,* 1987, vol. 47, 5715-5724

- **Ladner, R.C. ; Ley, A.C.** Novel frameworks as a source of high-affinity ligands. *Curr Opin Biotechnol,* 2001, vol. 12, 406-410

- Paramyxoviridae: The Viruses And Their Replication. **Lamb, R.A. ; Kolakofsky, D.** Virology. Lippincott Williams & Wilkins, 2001, 1305-1485

- **Lorence, R.M. ; Pecora, A.L. ; Major, P.P. ; Hotte, S.J. ; Laurie, S.A. ; Roberts, M.S. ; Groene, W.S. ; Bamat, M.K.** Overview of phase I studies of intravenous administration of PV701, an oncolytic virus. *Curr Opin Mol Ther,* 2003, vol. 5, 618-624

- **Mizukami, Y. ; Jo, W.S. ; Duerr, E.M. ; Gala, M. ; Li, J. ; Zhang, X. ; Zimmer, M.A. ; Iliopoulos, O. ; Zukerberg, L.R. ; Kohgo, Y.** Induction of interleukin-8 preserves the angiogenic response in HIF-1alpha-deficient colon cancer cells. *Nat Med,* 2005, vol. 11, 992-997

- **Nakaya, T. ; Cros, J. ; Park, M.S. ; Nakaya, Y. ; Zheng, H. ; Sagrera, A. ; Villar, E. ; Garcia-Sastre, A. ; Palese, P.** Recombinant Newcastle disease virus as a vaccine vector. *J Virol,* 2001, vol. 75, 11868-11873

- **Noonberg, S.B. ; Benz, C.C.** Tyrosine kinase inhibitors targeted to the epidermal growth factor receptor subfamily: role as anticancer agents. *Drugs,* 2000, vol. 59, 753-767

- **Nord, K. ; Gunneriusson, E. ; Nygren, P.A.** Ligands selected from combinatorial libraries of protein A for use in affinity capture of apolipoprotein A-1M and taq DNA polymerase. *J Biotechnol,* 2000, vol. 80, 45-54
- **Roffler, S.R. ; Wang, S.M. ; Chern, J.W. ; Yeh, M.Y ; Tung, E.** Antineoplastic glucuronide prodrug treatment of human tumor cells targeted with a monoclonal antibody-enzyme conjugate. *Biochem Pharmacol,* 1991, vol. 42, 2062-2065
- **Romer-Oberdorfer, A., Mundt, E. ; Mebatsion, T. ; Buchholz, U.J. ; Mettenleiter, T.C.** Generation of recombinant lentogenic Newcastle disease virus from cDNA. *J Gen Virol,* 1999, vol. 80 (11), 2987-2995
- Antibody guided enzyme nitrile therapy (Agent): in vitro cytotoxicity and in vivo tumor localisation. **Rowlinson-Busza, G. ; Bamias, A. ; Krausz, T. ; Epenetos, A.A.** Monoclonal Antibodies 2. Applications in Clinical Oncology. Chapman and Hall, 1992, 111-118
- **Russell, S.J.** RNA viruses as virotherapy agents. *Cancer Gene Ther,* 2002, vol. 9, 961-966
- **Salgaller, M.L.** Technology evaluation: bevacizumab, Genentech/Roche. *Curr Opin Mol Ther,* 2003, vol. 5, 657-667
- **Schnell, M.J. ; Mebatsion, T. ; Conzelmann, K.K.** Infectious rabies viruses from cloned cDNA. *Embo J,* 1994, vol. 13, 4195-4203
- **Schultz, U. ; Rinderle, C. ; Sekellick, M.J. ; Marcus, P.I. ; Staeheli, P.** Recombinant chicken interferon from Escherichia coli and transfected COS cells is biologically active. *Eur J Biochem.,* 1995, vol. 229, 73-76
- **Schwarz, H. ; Harlin, O. ; Ohnemus, A. ; Kaspers, B. ; Staeheii, P.** Synthesis of IFN-beta by virus-infected chicken embryo cells demonstrated with specific antisera and a new bioassay. *J Interferon Cytokine Res.,* 2004, vol. 24, 179-184
- **Sick, C. ; Schultz, U. ; Staeheli, P.** A family of genes coding for two serologically distinct chicken interferons. *J Biol Chem.,* 1996, vol. 271, 7635-7639
- **Sinkovics, J.G. ; Horvath, J.C.** Newcastle disease virus (NDV): brief history of its oncolytic strains. *J Clin Virol,* 2000, vol. 16, 1-15
- **Skerra, A.** Lipocalins as a scaffold. *Biochim Biophys Acta,* 2000, vol. 1482, 337-350
- **Staeheli, P. ; Puehler, F. ; Schneider, K. ; Gobel, T.W. ; Kaspers, B.** Cytokines of birds: conserved functions--a largely different look. *J Interferon Cytokine Res.,* 2001, vol. 21, 993-1010
- **Stojdl, D.F. ; Lichty, B.D. ; tenOever, B.R. ; Paterson, J.M. ; Power, A.T. ; Knowles, S. ; Marius, R. ; Reynard, J. ; Poliquin, L. ; Atkins, H.** VSV strains with defects in their ability to shutdown innate immunity are potent systemic anti-cancer agents. *Cancer Cell,* 2003, vol. 4, 263-275
- **Vogelstein, B. ; Kinzler, K.W.** Cancer genes and the pathways they control. *Nat Med,* 2004, vol. 10, 789-799
- **Waltenberger, J. ; Claesson-Welsh, L. ; Siegbahn, A. ; Shibuya, M. ; Heldin, C.H.** Different signal transduction properties of KDR and Flt1, two receptors for vascular endothelial growth factor. *J Biol Chem,* 1994, vol. 269, 26988-26995
- **Wang, S.M. ; Chern, J.W. ; Yeh, M.Y. ; Ng, J.C. ; Tung, E. ; Roffler, S.R.** Specific activation of glucuronide prodrugs by antibody-targeted enzyme conjugates for cancer therapy. *Cancer Res,* 1992, vol. 52, 4484-4491
- **Warren, R.S. ; Yuan, H. ; Matli, M.R. ; Gillett, N.A. ; Ferrara, N.** Regulation by vascular endothelial growth factor of human colon cancer tumorigenesis in a mouse model of experimental liver metastasis. *J Clin Invest,* 1995, vol. 95, 1789-1797
- **Zewe, M. ; Rybak, S.M. ; Dubel, S. ; Coy, J.F. ; Welschof, M. ; Newton, D.L. ; Little, M.** Cloning and cytotoxicity of a human pancreatic RNase immunofusion. *Immunotechnology,* 1997, vol. 3, 127-136
- **Zhao, H. ; Peeters, B.P.** Recombinant Newcastle disease virus as a viral vector: effect of genomic location of foreign gene on gene expression and virus replication. *J Gen Virol,* 2003, vol. 84, 781-788